# EUROPEAN PATENT APPLICATION

(11) **EP 1 908 835 A2**
(43) Date of publication of application: **09.04.2008**
(21) Application number: 07019198.6
(22) Date of filing: 16.07.2001
(51) Int. Cl.: C12N 15/12, C12N 15/62, C12N 15/63, C07K 14/47, C07K 14/72

(54) **Regulation of gene expression using single-chain, monomeric, ligand dependent polypeptide switches**

(30) Priority: 18.07.2000 US 619063
(62) Divisional of application: 01951681.4
(71) Applicant: Novartis AG, 4056 Basel (CH); Novartis Pharma GmbH, 1230 Vienna (AT); THE SCRIPPS RESEARCH INSTITUTE, La Jolla, CA 92037 (US)
(72) Inventor: Beerli, Roger, 8106 Adlikon (CH); Schopfer, Ulrich, 79539 Lörrach (DE); Barbas, Carlos F., Solana Beach, CA 92075 (US)
(74) Representative: Winter, Brandl, Fürniss, Hübner Röss, Kaiser, Polte Partnerschaft Patent- und Rechtsanwaltskanzlei

(57) **Abstract**

The invention relates to single chain, monomeric polypeptide gene switches. The gene switches include ligand binding domains and at least one functional domain. Preferred functional domains are DNA binding domains. The invention also relates to methods regulating gene function using these switches

## Description

### Technical Field of the Invention

The field of this invention is regulation of transcription. More particularly, the present invention pertains to polypeptides that can activate or repress transcription in a small molecule ligand-dependent manner.

### Background of the Invention

Designed transcription factors with defined target specificity and regulatory function provide invaluable tools for basic and applied research, and for gene therapy. Accordingly, the design of sequence-specific DNA binding domains has been the subject of intense interest for the last two decades. Of the many classes of DNA binding proteins studied, the modular Cys2 -His₂ zinc finger DNA binding motif has shown the most promise for the production of proteins with tailored DNA binding specificity. The novel architecture of this class of proteins provides for the rapid construction of gene-specific targeting devices. Polydactyl zinc finger proteins are most readily prepared by assembly of modular zinc finger domains recognizing predefined three-nucleotide sequences (See, e.g., Segal, D. J., Dreier, B., Beerli, R. R., and Barbas, C. F., III (1999) Proc. Natl. Acad. Sci. USA 96, 2758-2763; Beerli, R. R., Segal, D. J., Dreier, B., and Barbas, C. F., III (1998) Proc. Natl. Acad. Sci. USA 95, 14628-14633; and Beerli, R. R., Dreier, B., and Barbas, C. F., III (2000) Proc. Natl. Acad. Sci. USA 97, 1495-1500). Polydactyl proteins can be assembled using variable numbers of zinc finger domains of varied specificity providing DNA binding proteins that not only recognize novel sequences but also sequences of varied length. By combining six zinc finger domains, proteins have been produced that recognize 18 contiguous base pairs of DNA sequence, a DNA address sufficiently complex to specify any locus in the 4 billion-base pair human genome (or any other genome). Fusion of polydactyl zinc finger proteins of this type to activation or repression domains provides transcription factors that efficiently and specifically modulate the expression of both transgenes and endogenous genes (Beerli, R R., Segal, D. J., Dreier, B., and Barbas, C. F., III (1998) Proc. Natl. Acad, Sci. USA 95, 14628-14633; and Beerli, R. R., Dreier, B., and Barbas, C. P., III (2000) Proc. Natl. Acad. Sci. USA 97,1495-1500).

While the availability of designed transcription factors with tailored DNA binding specificities provides novel opportunities in transcriptional regulation, additional applications would be available to ligand-dependent transcription factors. Designer zinc finger proteins dependent on small molecule inducers would have a number of applications, both for the regulation of endogenous genes, and for the development of inducible expression systems for the regulation of transgenes. Natural transcription factors are regulated by a number of different mechanisms, including postranslational modification such as phosphorylation (Janknecht, R., and Hunter, T. (1997) EMBO j16, 1620-1627; Darnell, J. B., Jr. (1997) Science277, 1630-1635), or by ligand binding. The prototype ligand-activated transcription factors are members of the nuclear hormone receptor family, including the receptors for sex steroids or adrenocorticoids (Carson-Jurica, M. A., Schrader, W. T., and O'Malley, W. (1990) Endocrine Reviews11, 201-220; Evans, R. M. (1988) Science240, 889-895). These receptors are held inactive in the absence of hormone, by association with a number of inactivating factors including hsp90 (Pratt, W. B., and Toft, D. O. (1997) Endocrine Rev. 18, 306-360). Upon ligand binding, nuclear hormone receptors dissociate from the inactivating complex, dimerize, and become able to bind DNA and activate transcription (Carson-Jurica, M. A., Schrader, W. T., and O'Malley, W. (1990) Endocrine Reviews 11, 201-220; Evans, R. M. (1988) Science 240, 889-89512-14; and Pratt, W. B., and Toft, D. O. (1997) Endocrine Rev. 18, 306-360). Significantly, not only hormone binding but also inactivation and dimerization functions reside within the ligand binding domain (LBD) of these proteins (Brato, M. (1989) Cell 56, 335-344). This fact has been exploited experimentally and steroid hormone receptor LBDs have found wide use as tools to render heterologous proteins hormone-dependent.

In particular, the estrogen receptor (ER) LBD has been used to render the functions of c-Myc (Eil*ers, M., Picard, D., Yamamoto, K. R., and Bishop, J. M. (1989) Nature 340, 66-68), c-Fos (Superti-Furga, G., Bergers, G., Picard, D., and Busslinger, M. (1991) Proc. Natl. Acad. Sci. USA 88, 5114-5118), and even the cytoplasmic kinase c-Raf (. Samuels, M. L., Weber, M. J., Bishop, J. M., and McMahon, M. (1993) Mol. Cell. Biol. 13, 6241-6252) hormone-dependent. To develop an inducible expression system for use in basic research and gene therapy, the availability of ligand-dependent transcriptional regulators is a prerequisite. Preferentially, these regulators would be activated by a small molecule inducer with no other biological activity, bind specific sequences present only in the target promoter, and have low immunogenicity. A number of ligand-regulated artificial transcription factors have been generated by various means, using functional domains derived from either prokaryotes (Gossen, M., and Bujard, H. (1992) Proc. Natl. Acad. Sci. USA 89, 5547-5547-5551 20. Gossen, M., Freundlieb, S., Bender, G., Müller, G., Hillen, W., and Bujard, H. (1995) Science 268,1766-1769 21. Labow, M. A., Baim, S. B., Shenk, T., and Levine, A. J. (1990) Mol. Cell. Biol. 10, 3343-3356 22. Baim, S. B., Labow, M. A., Levine, A. J., and Shenk, T. (1991) Proc. Natl. Acad. Sci. USA 88, 5072-5076) or eukaryotes (Christopherson, K. S., Mark, M. R., Bajaj, V., and Godowski, P. J. (1992) Proc. Natl. Acad. Sci. USA 89, 6314-6318 24. No, D., Yao, T.-P., and Evans, R. M. (1996) Proc. Natl. Acad. Sci. USA 93, 3346-3351 25. Wang, Y., O'Malley, B. W., Jr., Tsai, S., and O'Malley, B. W. (1994) Proc. Natl. Acad. Sci. USA 91, 8180-8184 Beerli *et al. -*35-26. Wang, Y., Xu, J., Pierson, T., O'Malley, B. W., and Tsai, S. Y. (1997) Gene Therapy 4, 432-441 27. Braselmann, S., Graninger, P., and Busslinger, M. (1993) Proc. Natl. Acad. Sci. USA 90, 1657-1661 28. Louvion, J. F., Havaux-Copf, B., and Picard, D. (1993) Gene 131,129-134 29. Rivera, V. M; Clackson, T., Natesan, S., Pollock, R., Amara, J. F., Keenan, T., Magari, S. R., Phillips, T., Courage, N. L., Cerasoli, F., Jr., Holt, D. A., and Gilman, M. (1996) Nature. Med. 2, 1028-1032).

Of the functional domains derived from eukaryotic proteins, nuclear hormone receptor LBDs have been the most widely used. In particular, regulators based on the Ga14 DNA binding domain (DBD) fused to a human ER (Braselmann, S., Graninger, P., and Busslinger, M. (1993) Proc. Natl. Acad. Sci. USA 90, 1657-1661; Louvion, J. F., Havaux-Copf, B., and Picard, D. (1993) Gene 131, 129-134) or progesterone receptor (PR) LBD; (Wang, Y., O'Malley, B. W., Jr., Tsai, S., and O'Malley, B. W. (1994) Proc. Natl. Acad. Sci. USA 91, 8180-8184; Wang, Y., Xu, J., Pierson, T., O'Malley, B. W., and Tsai, S. Y. (1997) Gene Therapy 4, 432-441), as well as the ecdysone-inducible system based on the *Drosophila* ecdysone receptor (EcR) and the mammalian retinoid X receptor (RXR) (Christopherson, K. S., Mark, M. R., Bajaj, V., and Godowski, P. J. (1992) Proc. Natl. Acad. Sci. USA 89, 6314-6318; No, D., Yao, T.-P., and Evans, R. M. (1996) Proc. Natl. Acad. Sci. USA 93, 3346-3351) have been described. Compared to the heterodimeric EcR/RXR system, regulators based on the ER and PR LBDs have the important advantage that they function as homodimers and require the delivery of only one cDNA. However, while ecdysone has no known biological effect on mammalian cells, estrogen and progesterone will elicit a biological response in cells or tissues that express the endogenous steroid receptors. With the availability of a mutated ER and a truncated PR LBDs that have lost responsiveness to their natural ligands but not to synthetic antagonists such as 4-hydroxytamoxifen (4-OHT) (Littlewood, T. D., Hancock, D. C., Danielian, P. S., Parker, M. G., and Evan, G. I. (1995) Nucl. Acids Res. 23, 1686-1690) or RU486 (Vegeto, E., Allan, G. F., Schrader, W. T., Tsai, M.-J., McDonnell, D. P., and O'Malley, B. W. (1992) Cell69, 703-713), respectively, this is no longer of great concern. Thus, steroid hormone receptor LBD-based inducible expression systems can be developed that function independently of the endogenous steroid receptors. To date, this has been shown for the PR LBD through the development of an RU486-inducible expression system based on the Ga14 DBD (Wang, Y., Q'Malley, B. W., Jr., Tsai, S., and O'Malley, B. W. (1994) Proc. Natl. Acad Sci. USA 91, 8180-8184; Wang, Y., Xu, J., Pierson, T., O'Malley, B. W., and Tsai, S. Y. (1997) Gene Therapy 4, 432-441). An inducible expression system based on a point-mutated (G525R) ER LBD (Littlewood, T. D., Hancock, D. C., Danielian, P. S., Parker, M. G., and Evan, G.L. (1995) Nacl. Acids Res. 23, 1686-1690) that has lost the responsiveness to estrogen but not the antagonist 4-OHT has not been described to date. Designed zinc finger proteins have a number of advantages as compared to other DBDs, including the one derived from Ga14, since the ability to engineer DNA binding specificities allows ligand-dependent regulators to be directed to any desired artificial or natural promoter. Here we explore the utility of fusion proteins between designed zinc finger proteins and nuclear hormone receptor LBDs for the inducible control of gene expression.

### Brief Summary of the Invention

In one embodiment, the present invention provides a non-naturally occurring polypeptide that contains two ligand binding domains operatively linked to each other and a first functional domain operatively linked to one of the ligand binding domains. The ligand binding domains are preferably covalently linked to each other. More preferably, the two binding domains are covalently linked by means of a peptide linker that contains from about 10 to about 40 amino acid residues, preferably from about 15 to about 35 amino acid residues and, more preferably from about 18 to about 30 amino acid residues.

In one embodiment, the ligand binding domains are derived from nuclear hormone receptors. The ligand binding domains can be derived from the same or different nuclear hormone receptors. Exemplary and preferred nuclear hormone receptors are steroid hormone receptors such as an estrogen receptor, a progesterone receptor, an ecdysone receptor and a retinoid X receptor.

The first functional domain can be any domain that alters the function or activity of a target nucleotide. In one embodiment, the first functional domain is a nucleotide binding domain. Preferably, the nucleotide binding domain is a DNA binding domain. The DNA binding domain preferably contains at least one zinc finger DNA binding motif, more preferably from two to twelve zinc finger DNA binding motifs and, even more preferably from three to six zinc finger DNA binding motifs. In one embodiment, the zinc finger DNA binding motifs specifically bind to a nucleotide sequence of the formula (GNN)₁₋₆, where G is guanidine and N is any nucleotide. In another embodiment, the first functional domain is a transcriptional regulating domain such as a transcription activation domain or a transcription repression domain.

In still another embodiment, the polypeptide gene switch contains a second functional domain. In accordance with this embodiment, a preferred first functional domain is a nucleotide binding domain and the second functional domain is a transcriptional regulating domain.

In one embodiment, a polypeptide of this invention includes (a) a DNA binding domain having from three to six zinc finger DNA binding motifs; (b) a first ligand binding domain derived from a retinoid X receptor operative linked to the DNA binding domain, a second ligand binding domain derived from an ecdyzone receptor linked to the first ligand binding domain with a peptide spacer of from 18 to 36 amino acid residues; and (c) a transcription regulating domain operatively linked to the second binding domain.

In still another embodiment, a polypeptide gene switch includes (a) a DNA binding domain having from three to six zinc finger DNA binding motifs; (b) a first ligand binding domain derived from a progesterone receptor operatively linked to the DNA binding domain, a second ligand binding domain derived from a progesterone receptor linked to the first ligand binding domain with a peptide spacer of from 18 to 36 amino acid residues; and (c) a transcription regulating domain operatively linked to the second ligand binding domain.

In another aspect, the present invention provides polynucleotides that encode a polypeptide gene switch of the invention, expression vectors containing such polynucleotides and cells containing such nucleotides.

Another aspect of this invention provides a process of regulating the function of a target nucleotide that contains a defined sequence. The process includes the step of exposing the target nucleotide to a polypeptide of this invention in the presence of a ligand that binds at least one of the ligand binding domains of the polypeptide. In a related aspect, the present invention provides a process for regulating transcription (e.g., expression) of a target nucleotide (e.g., gene). In accordance with that process a target nucleotide that contains a defined sequence is exposed to a polypeptide of this invention in the presence of a ligand that binds to at least one of the ligand binding domains of that polypeptide. The polypeptide contains a nucleotide binding domain that specifically binds to the defined sequence in the target nucleotide. Where the polypeptide gene switch contains a transcription repression domain, regulating is repression. Where the polypeptide gene switch contains a transcription activation domain, regulating is activation.

### Brief Description of the Drawings

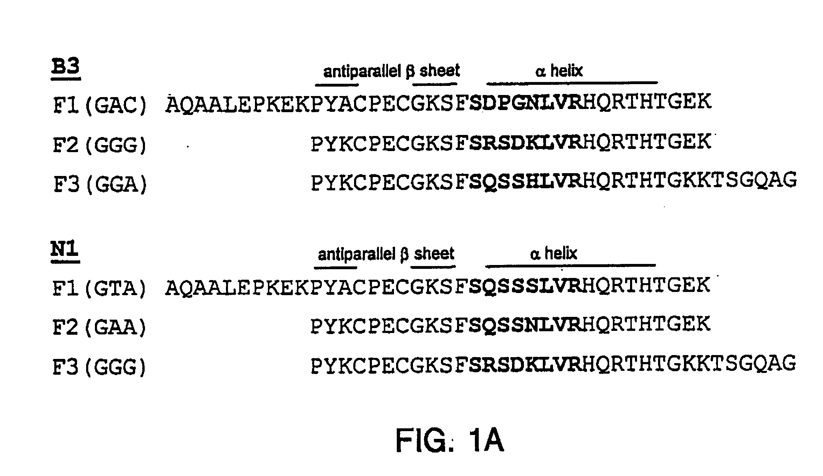

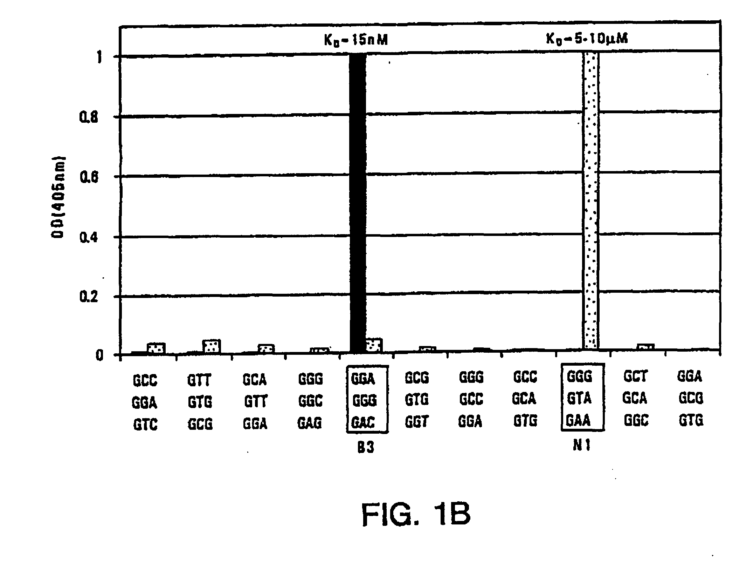

In the drawings that form a portion of the specification
**Figure 1** shows generation of designed zinc finger proteins with novel DNA binding specificity. A, amino acid sequence of the three-finger proteins B3 and N1. DNA recognition helix positions -2 to 6, shown in bold print, were grafted into the framework of the three finger protein Sp1C. The location of the antiparallel β sheets and the α helices, structural hallmarks of zinc finger domains, are as indicated. DNA binding specificity of each finger is show on the left. F1-3, Finger 1-3. *B,* ELISA analysis of DNA binding specificity. Zinc finger proteins were expressed in *E. coli* as MBP fusions and purified. Specificity of binding was analyzed by measuring binding to immobilized biotinylated hairpin oligonucleotides containing the indicated 5'-(GNN)₃-3'sequences. Black bars, B3; gray bars, N1. The maximal signals were normalized to 1. The K_{D} value for binding to the specific target sequence was measured by electrophoretic mobility shift assay and is labeled on top of the corresponding bars.

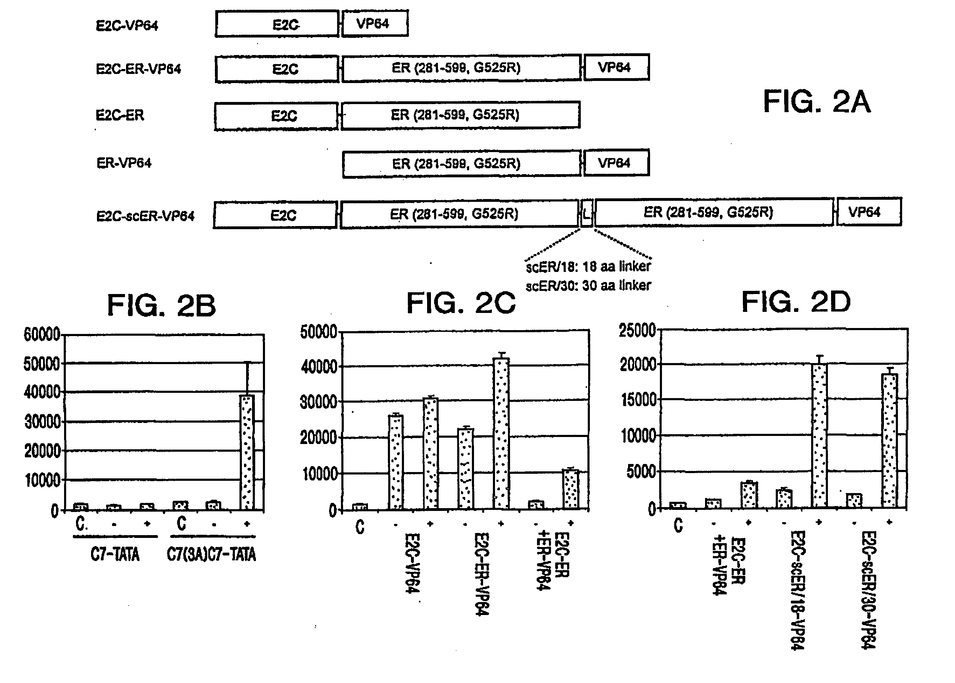

**Figure 2** shows regulation of gene expression by hormone-dependent, single-chain ER fusion constructs. A, structure of ER fusion proteins. E2C, six finger protein; L, flexible peptide linker. *B*, fusion proteins with a single ER-LBD bind as dimers. HeLa cells were cotransfected with a C7-ER-VP64 expression vector, and the indicated TATA luciferase reporter plasmids carrying either one or two C7 binding sites. 24 h after transfection, cells were either left untreated (-), or 100 nM 4-OHT was added (+). Luciferase activity in total cell extracts was measured 48 h after transfection. Each bar represents the mean value (+/- SD) of duplicate measurements. C, control plasmid pcDNA3 that does not express a fusion protein. *C*, *D*,-regulation of transcription through a single binding site by fusion proteins with two ER-LBDs. HeLa cells were cotransfected with the indicated expression vectors and the E2C-TATA-luciferase reporter plasmid, carrying a single E2C binding site upstream of a TATA box. 4-OHT induction and measurement of luciferase activity was carried out as described in *B.*

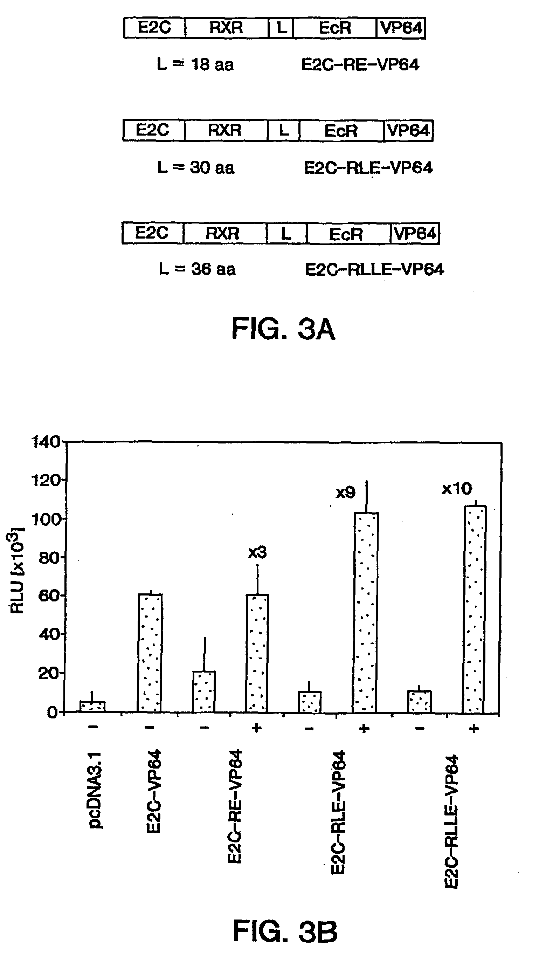

**Figure 3** shows regulation of gene expression by hormone-dependent, single-chain RXR/EcR fusion constructs. *A,* structure of single-chain RXR/EcR fusion proteins. *B*, regulation of transcription through a single binding site. HeLa cells were cotransfected with the indicated expression vectors and the E2C-TATA-luciferase reporter plasmid, carrying a single E2C binding site. 24 h after transfection, cells were either left untreated (-), or 5 µM Ponasterone A was added (+). Luciferase activity in total cell extracts was measured 48 h after transfection. Each bar represents the mean value (+/- SD) of duplicate measurements. pcDNA3.1, control plasmid that does not express a fusion protein.
**Figure 4** shows the nucleotide (SEQ ID NO: 31) and amino acid residue sequence (SEQ ID NO: 32) of zinc finger binding domain B3B.
**Figure 5** shows the nucleotide (SEQ ID NO: 33) and amino acid residue sequence (SEQ ID NO: 34) of zinc finger binding domain 2C7.
**Figure 6** shows the nucleotide (SEQ ID NO: 35) and amino acid residue sequence (SEQ ID NO: 36) of zinc finger binding domain B3C2.
**Figure 7** shows the nucleotide (SEQ ID NO: 37) sequence of repression domain (KRAB-A)₂.
**Figure 8** shows the nucleotide (SEQ ID NO: 38) sequence of repression domain (SID)₂.
**Figure 9** shows the nucleotide (SEQ ID NO: 39) and amino acid residue sequence (SEQ ID NO: 40) of polypeptide B2C-ER-L-ER-VP64.
**Figure 10** shows the nucleotide (SEQ ID NO: 41) and amino acid residue sequence (SEQ ID NO: 42) of polypeptide E2C-ER-LL-ER-VP64.

### Detailed Description of the invention

### I. The Invention

The present invention provides polypeptide gene switches, polynucleotides that encode such polypeptides, expression vectors that contain such polynucleotides, cells that contain such expression vectors or polynucleotides and processes for regulating target nucleotide function using such polypeptides, polynucleotides and expression vectors. Unlike existing gene switches that contain a single ligand binding domain together with a DNA binding domain and/or a transcriptional regulating domain, polypeptide gene switches of the present invention contain two ligand binding domains. Upon binding of the ligand, an intramolecular configuration change occurs that allows for alignment of the functional domains to the target gene of interest. An advantage of the present gene switches, therefore, over existing gene switches is the need for only a single molecular switch and a single expression vector for production of that switch.

### II. Polypeptides

A polypeptide gene switch of the present invention includes at least three components: two ligand binding domains (LBDs) and a first functional domain (FD-1). The ligand binding domains are operatively linked to the first functional domain such that the polypeptide, in the presence of a defined ligand that binds to at least one of the ligand binding domains, can alter the function of nucleotide. The domains can be arranged in any order. As shown below, the ligand binding domains can be situated in either the amino-or carboxyl-terminal direction from the first functional domain.

| | |
|---|---|
| LBDs | FD-1 |

| | |
|---|---|
| FD-1 | LBDs |

A polypeptide of this invention is non-naturally occurring. As used herein, the term "non-naturally occurring" means, for example, one or more of the following: (a) a peptide comprised of a non-naturally occurring amino acid sequence; (b) a peptide having a non-naturally occurring secondary structure not associated with the peptide as it occurs in nature; (c) a peptide which includes one or more amino acids not normally associated with the species of organism in which that peptide occurs in nature; (d) a peptide which includes a stereoisomer of one or more of the amino acids comprising the peptide, which stereoisomer is not associated with the peptide as it occurs in nature; (e) a peptide which includes one or more chemical moieties other than one of the natural amino acids; or (f) an isolated portion of a naturally occurring amino acid sequence (e.g., a truncated sequence). A polypeptide of this invention exists in an isolated form and purified to be substantially free of contaminating substances. A polypeptide is synthetic in nature. That is, the polypeptide is isolated and purified from natural sources or made *de novo* using techniques well known in the art.

### A. Ligand Binding Domain (LBD)

Each LBD is an amino acid residue sequence that is capable of and binds a particular ligand. Binding of the ligand to the LBD alters the conformation/funetion of the polypeptide and allows for regulating a function of a target nucleotide. In the absence of ligand, the gene switch does not work to alter nucleotide function. At least one of the LBDs is capable of binding and binds a particular ligand. Both LBDs can bind a particular ligand. Thus, the LBDs can be the same or different. Preferred LBDs are derived from nuclear hormone receptors such as steroid hormone receptors.

Exemplary and preferred steroid receptors that can serve as the source of ligand binding domains include the estrogen receptor (ER), progesterone receptor (PR), glucocorticoid-α receptor, glucocorticoid-β receptor, mineralocorticoid receptor, androgen receptor, thyroid hormone receptor, retinoic acid receptor (RAR), retinoid X receptor (RXR), Vitamin D receptor, CDUP-TF receptor, ecdysone receptor (EcR), Nurr-1 receptor and orphan receptors. A preferred EcR is derived either from *Drosophila melanogaster* (DE) or *Bombyx* (BE).

As is well known in the art, steroid hormone are composed of a DNA binding domain and a ligand binding domain. The DNA binding domain contains the receptor regulating sequence and binds DNA and the ligand binding domain binds the specific biological compound (ligand) to activate the receptor. The term "ligand" refers to any compound which activates the receptor, usually by interaction with (binding) the ligand binding domain of the receptor. However, ligands also include compounds that activate the receptor without binding. Where used in a polypeptide gene switch of the present invention, it is preferred that the ligand receptor domain be modified from its naturally occurring ligand, a ligand other than the naturally occurring ligand (e.g. steroid hormone). Means of altering or derivatizing naturally occurring nuclear hormone receptor ligand binding domains to alter the binding specificity are well known in the art (See. e.g. United States Patent Nos. 5,874,534 and 5,599,904 the disclosures of which are incorporated herein by reference). Similarly, means for altering the estrogen receptor to change its bind affinity have reported [See, e.g. Littlewood et al., Nucleic Acids Res., 3(10):1686-1690,1995].

The term "naturally occurring ligand" refers to compounds that are normally not found in animals or humans and which bind to the ligand binding domain of a receptor. The ligand can also be a "non-native ligand", a ligand that is not naturally found in the specific organism (man or animal) in which gene therapy is contemplated. For example, certain insect hormones such as ecdysone are not found in humans. This is an example of a non-native hormone to the animal or human.

Examples of non-natural ligands, and-honnones and non-native ligands include the following: 11β-(4-dimemylaminophenyl)-17β-hydroxy-17α-propinyl-4,9-e stradieno-3-one (Ru38486 or Mifepestone); 11β-(4-dimethylaminophenyl)-17α-hydroxy-17β-(3-hydroxypropyl)-13α-methyl-4,9-gonadiene-3-one (ZK98299 or Onapristone); 11β-(4-acetylphenyl)-17β-hydroxy-17α-(1-propinyl)-4,9-estradiene-3-one (ZK112993); 11β-(4-dimenthylaminophenyl)-17β-hydroxy-17α-(3-hydroxy-1 (Z)-propenyl-estra-4,9-diene-3-one (ZK98734); (7β11β17β)-11-(4-dimethylaminophenyl)-7-methyl-4',5' dihydrospiroy'ester-4,9-diene-17,2'(3'H)-furanl-3-one (Org31806); (11β,14β,17α)-4',5'-dihydro-11-(4-dimethylaminophenyl)y'spirostra-4,9-diene-17,2'(3'H)-furan!-3-one (Org31376); 5-alpha-pregnane-3,2-dione. Additional non-natural ligands include, in general, synthetic non-steroidal estrogenic or anti-estrogenic compounds, broadly defined as selective estrogen receptor modulators (SERMS). Exemplary compounds include, but are not limited to, tamoxifen and raloxifen,

Exemplary and preferred ligands for use with various ligand binding domains are (1) EcR: Ponasterone a, Muristerone A, GS-E (Invitrogen), Tebufenocide; (2) ER: estrogen antagonists such as 4-hydroxy-tamoxifen, ICI 164384, RU 54876, Raloxifene; and (3) PR: progesterone antagonists such as RU 486, RU 38486, and Onapristone.

An especially preferred LBD derived from a progesterone receptor comprises amino acid residues 645-914 from the human progesterone receptor. An exemplary LBD derived from an estrogen receptor comprises amino acid residues 282-599 from the mouse G225R mutant.

The two LBDs are separated be an amino acid residue sequence linker that contains from about 10 to about 50 amino acid residues. Preferably, the spacer contains from about 15 to about 40 amino acid residues and, more preferably, from about 18 to about 35 amino acid residues. Exemplary and preferred spacers contain 18 (L), 30 (LL), or 36 (LLL) amino acid residues.

### B. Functional Domains

A second component of a present polypeptide is a functional domain. As used herein, the term "functional domain" and it's grammatical equivalents, means an amino acid residue sequence that binds to, alter the structure of, and/or alters the function of, a nucleotide. Exemplary such functional domains include nucleotide binding domains, transcriptional regulating domains (e.g. transcription activation domains and transcription repression domains) and domains having nuclease activity. Such domains are well known in the art.

### 1. Nucleotide Binding Domains

A functional domain of a polypeptide can be a nucleotide binding domain: a sequence of amino acid residues that recognize and bind to a defined nucleotide sequence. The target nucleotide sequence can be an RNA sequence or, preferably, a DNA sequence. Amino acid residue sequences that recognize and bind to defined DNA sequences are well known in the art (e.g., GAL4). Any such DNA binding peptide can be used as a DNA binding domain of a polypeptide gene switch of this invention. It is preferred, however, that the DNA binding domain of a present gene switch be one or more DNA binding zinc finger motifs. Such zinc finger DNA binding motifs are well known in the art (See. e.g., PCT Patent Application Nos. WO95/19421 and WO 98/54311, the disclosures of which are incorporated herein by reference). A DNA binding domain of a polypeptide gene switch of this invention, thus, preferably includes a multiple finger, polydactyl, zinc finger peptide that is designed to bind specific nucleotide target sequences.

The present disclosure is based on the recognition of the structural features unique to the Cys₂-His₂ zinc finger domain consist of a simple ββα fold of approximately 30 amino acids in length. Structural stability of this fold is achieved by hydrophobic interactions and by chelation of a single zinc ion by the conserved Cys₂-His₂ residues (Lee, M.S., Gippert, G.P., Soman, K.V., Case, D.A. & Wright, P.E. (1989) Science 245, 635-637). Nucleic acid recognition is achieved through specific amino acid side chain contacts originating from the α-helix of the domain, which typically binds three base pairs of DNA sequence (Pavletich, N. P. & Pabo, C.O. (1991) Science 252, 809-17, Elrod-Erickson, M., Rould, M.A., Nekludova, L. & Pabo, C.O. (1996) Structure 4, 1171-1180). Unlike other nucleic acid recognition motifs, simple covalent linkage of multiple zinc finger domains allows the recognition of extended asymmetric sequences of DNA.

Studies of natural zinc finger proteins have shown that three zinc finger domains can bind 9 bp of contiguous DNA sequence (Pavletich, N.P. & Pabo, C.O. (1991) Science 252, 809-17., Swimoff, A.H. & Milbrandt, J. (1995) Mol. Cell. Biol. 15, 2275-87). Whereas recognition of 9 bp of sequence is insufficient to specify a unique site within even the small genome of *E.coli,* polydactyl proteins containing six zinc fingers domains can specify 18-bp recognition (Liu, Q., Segal, D.J., Ghiara, J.B. & Barbas III, C.F. (1997) Proc. Natl. Acad. Sci. USA 94,5525-5530). With respect to the development of a universal system for gene control, and 18-bp address can be sufficient to specify a single site within all known genomes. And their efficacy in gene activation and repression within living human cells has recently been shown (Liu, Q., Segal, D.J., Ghiara, J.B. & Barbas III, C.P. (1997) Proc. Natl. Acad. Sci. USA 94, 5525-5530).

The zinc finger-nucleotide binding peptide domain can be derived or produced from a wild type zinc finger protein by truncation or expansion, or as a variant of the wild type-derived polypeptide by a process of site directed mutagenesis, or by a combination of the procedures. The term "truncated" refers to a zinc finger-nucleotide binding polypeptide that contains less that the full number of zinc fingers found in the native zinc finger binding protein or that has been deleted of non-desired sequences. For example, truncation of the zinc finger-nucleotide binding protein TFIIIA, which naturally contains nine zinc fingers, might be a polypeptide with only zinc fingers one through three. Expansion refers to a zinc finger polypeptide to which additional zinc finger modules have been added. For example, TFIIIA may be extended to 12 fingers by adding 3 zinc finger modules from more than one wild type polypeptide, thus resulting in a "hybrid" zinc finger-nucleotide binding polypeptide.

The term "mutagenized" refers to a zinc finger derived-nucleotide binding polypeptide that has been obtained by performing any of the known methods for accomplishing random or site-directed mutagenesis of the DNA encoding proteins. For instance, in TFIIIA, mutagenesis can be preformed to replace non-conserved residues in one or more of the repeats of the consensus sequence. Truncated zinc finger-nucleotide binding proteins can also be mutagenized. Examples of known zinc finger-nucleotide binding proteins can also be mutagenized. Examples of known zinc finger-nucleotide binding polypeptides that can be truncated, expanded, and/or mutagenized according to the present invention in order to inhibit the function of a nucleotide sequence containing a zinc finger-nucleotide binding motif includes TFIIIA and zif268. Other zinc finger-nucleotide binding proteins will be known to those of skill in the art.

A zinc finger DNA binding domain can be make using a variety of standard techniques well known in the art. Phage display libraries of zinc finger proteins were created and selected under conditions that favored enrichment of sequence specific proteins. Zinc finger domains recognizing a number of sequences required refinement by site-directed mutagenesis that was guided by both phage selection data and structural information.

A DNA binding domain used in a polypeptide of this invention is preferably a zinc finger-nucleotide binding peptide that binds to a (GNN)₁₋₆ nucleotide sequence. Zinc fingers that bind specifically to (GNN)₁₋₆ have been described in United States Patent Application Serial Number 09/173,941, filed October 16,1998 (the disclosure of which is incorporated herein by reference).

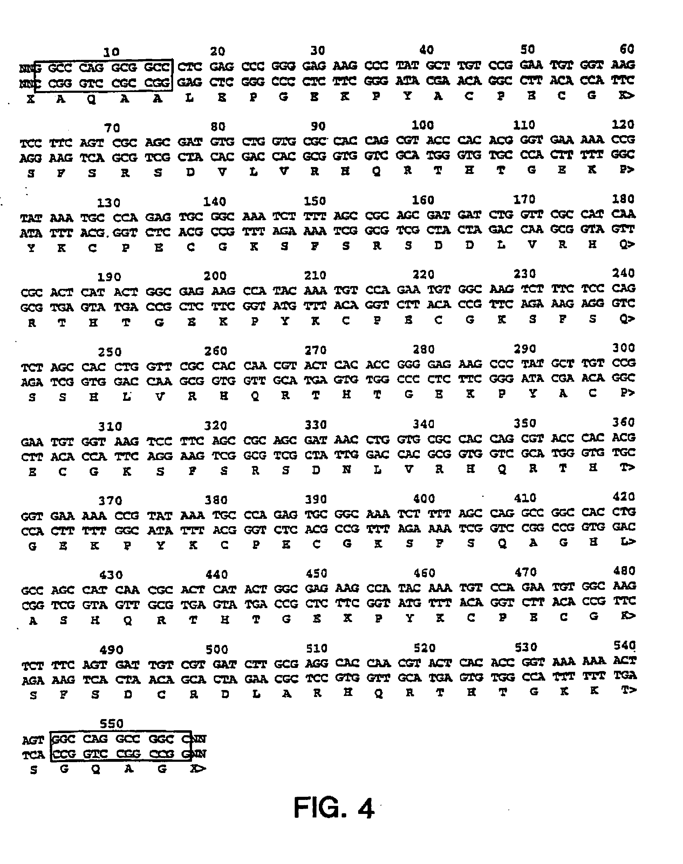

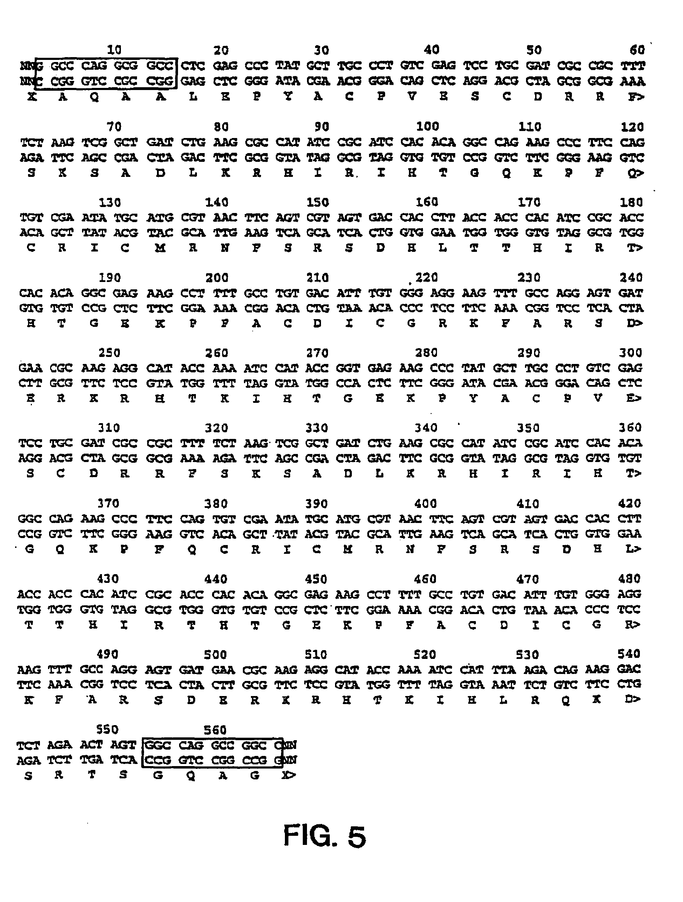

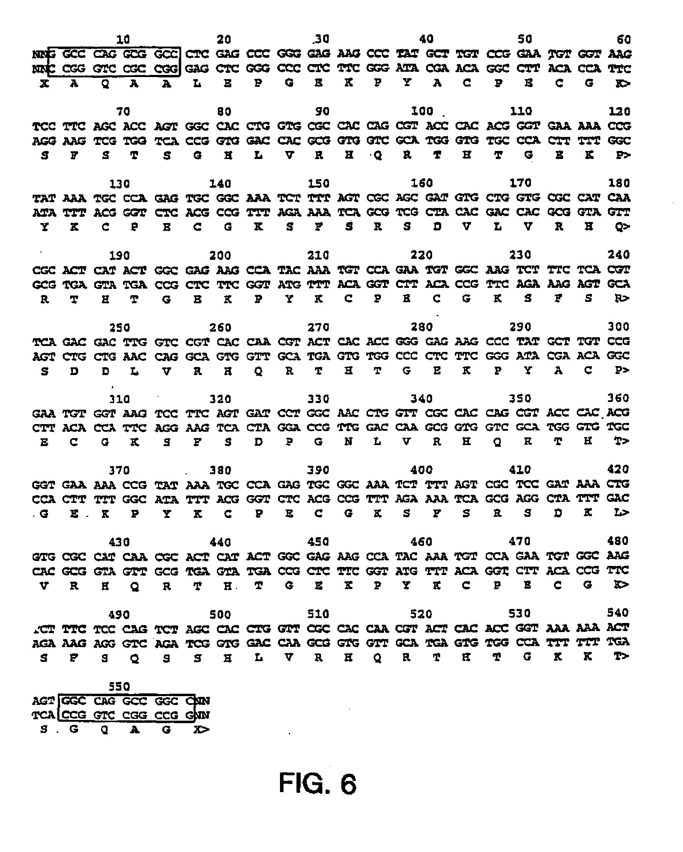

Exemplary and preferred zinc finger DNA binding domains are designated herein as E2C, C7, B3B, 2C7, B3C2 and N1. A detailed description of the preparation of polypeptide gene switches containing zinc finger DNA binding domains can be found hereinafter in the Examples. The amino acid residue and encoding nucleotide sequences for B3B, 2C7 and B3C2 are shown in FIGs. 4-6, respectively.

### 2. Transcription Regulating Domains

A transcription regulating domain refers to a peptide, which acts to activate or repress transcription of a target nucleotide (e.g., gene). Transcriptional activation domains are well known in the art (See, e.g., Seipel et al., (1992) EMBO J., 11:4961-4968). Exemplary and preferred transcription activation domains include VP16, TA2, VP64, STAT6, relA, TAF-1, TAF-2, TAU-1 and TAU-2. Especially preferred activation domains for use in the present invention are VP16 and VP64. Means for linking VP16 and VP64 to ligand binding domains are set forth hereinafter in the Examples.

Transcriptional repressor domains are also well known in the art. Exemplary and preferred such transcriptional repressors are ERD, KRAB, SID, histone deacetylase, DNA, methylase, and derivatives, multimers and combinations thereof such as KRAB-ERD, SID-ERD, (KRAB)₂, (KRAB)₃, KRAB-A, (KRAB-A)₂, (SID)₂, (KRAB-A)-SID and SID-(KRAB-A). A first repressor domain can be prepared using the Krη1ppel-asociated box (KRAB) domain (Margolin *et al.,* 1994). This repressor domain is commonly found at the N-terminus of zinc finger proteins and presumably exerts its repressive activity on TATA-dependent transcription in a distance- and orientation-independent manner, by interacting with the RING finger protein KAP-1. One can utilize the KRAB domain found between amino acids 1 and 97 of the zinc finger protein KOX1. Finally, to explore the utility of histone deacetylation for repression, amino acids 1 to 36 of the Mad mSIN2 interaction domain (SID) can be fused to another domain (Ayer *et al.,* 1996). This small domain is found at the N-terminus of the transcription factor Mad and is responsible for mediating its transcriptional repression by interacting with mSIN3, which in turn interacts the co-repressor N-CoR and with the histone deacetylase mRPD1.

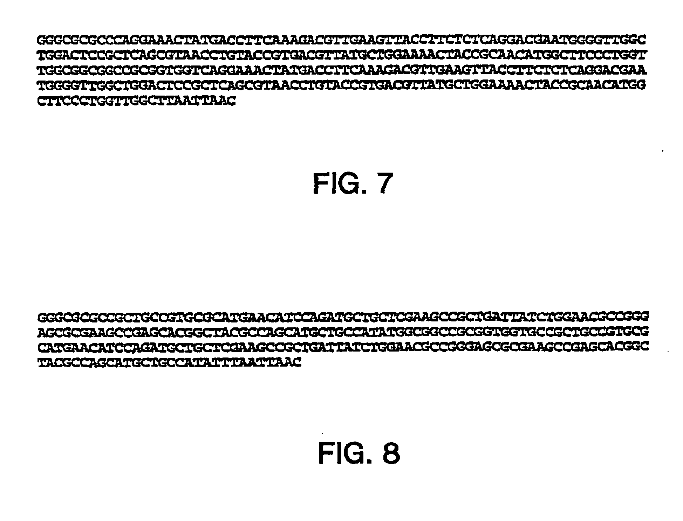

The amino acid residue and nucleotide encoding sequences of preferred transcriptional repression domains (KRAB-A)₂ and (SID)₂ are shown in FIGs 7 and 8, respectively. Means for linking repression domains to ligand binding domains as well as exemplary polypeptide gene switches containing repression domains are set forth hereinafter in the Examples.

### 3. Polypeptide Gene Switches

A polypeptide of this invention, in one embodiment, comprises two ligand binding domains and a first functional domain. In another embodiment, a polypeptide gene switch comprises two ligand binding domains, a first functional domain and a second functional domain. These domains can exist in any order as shown below.

In a preferred embodiment the two ligand binding domains (LBDs) are located directly adjacent to one another, ie. they are "serially connected" within the monomeric polypeptide gene switch of the invention and are not separated by a functional domain of the invention. The serially connected LBDs may be separated from one another by a linker molecule, such as for example a polypeptide linker molecule.

In a preferred embodiment the two LBDs are located between two functional domains (FDs) of the invention, wherein one functional domain is a Transcription Regulating Domain (TRD) and the other functional domain is a Nucleotide Binding Domain (NBD).

In one particularly preferred embodiment the monomeric polypeptide gene switch of the invention consists of two FDs and two LBDs in the sequential order FD-1 / LBD-1 / LBD-2 / FD-2. Preferredly, in this embodiment, one functional domain is a TRD and the other functional domain is a NBD.

Preferredly, the NBD employed in the monomeric polypeptide gene switch of the invention includes 6 zinc finger binding motifs. As further described in the examples hereinbelow, a 6 zinc finger NBD employed in a monomeric polypeptide gene switch allows for the recognition of a unique 18bp nucleic acid sequence, which may be symmetric or asymmetric.

| | | |
|---|---|---|
| LBDs | FD-1 | FD-2 |

| | | |
|---|---|---|
| FD-1 | FD-2 | LBDs |

| | | |
|---|---|---|
| FD-1 | LBDs | FD-2 |
| | | |
| FD-2 | LBDs | FD-1 |
| | | |
| FD-2 | FD-1 | LBDs |

A wide variety of polypeptide gene switches have been made. Exemplary such gene switches include (see above for definition of terms):
Gene Switches Using RXR, E2C, and Activation Domains
E2C-RXR-L-DB-VP64, E2C-RXR-LL-DE-VP64, B2C-RXR-LLL-DB-VP64, B2C-RXR-L-BE-VP64, E2C-RXR-LL-BE-VP64, BZC-RXR-LLL-BE-VP64, E2C-RXR-L-DE-VP16, B2C-RXR-LL-DE-VP16, E2C-RXR-LLL-DE-VP16, E2C-RXR-L-BE-VP16, E2C-RXR-LL-BE-VP16, E2C-RXR LLL-BE-VP16;
Gene Switches Using RXR, 2C7, and Activation Domains
2C7-RXR-L-DE-VP64, 2C7-RXR-LL-DE-VP64, 2C7-RXR-LLL-DE-VP64, 2C7-RXR-L-BE-VP64,2C7-RXR-LL-BE-VP64.2C7-RXR-UL-BE-VP64, 2C7-RXR-L-DE-VP16, 2C7-RXR-IL-DE-VP16, 2C7-RXR-LLL-DE-VP16,2C7-RXR-L-BE-VP16,2C7-RXR-LL-BE-VP16, E2C-RXR-LLL-BE-VP16;
Gene Switches Using RXR B3B, and Activation Domains
B3B-RXR-L-DE-VP64, B3B-RXR-LL-DE-VP64, B3B-RXR-LLL-DE-VP64, B3B 7-RXR-L-BE-VP64, B3B 7-RXR-LL-BE-VP64, B3B-RXR-LLL-BE-VP64, B3B-RXR-L-DE-VP16, B3B-RXR LL-DE-VP16, B3B-RXR-LLL-DE-VP16, B3B-RXR-L-BE-VP16, B3B-RXR-LL-BE-VP16, B3B-RXR-LLL-BE-VP16;
Gene Switches Using RXR, B3C2, and Activation Domains
B3C2-RXR-L-DE-VP64., B3C2-RXR-LL-DE-VP64, B3C2-RXR-LLL-DE-VP64, B3C2-RXR-L-BE-VP64, B3C2-RXR-LL-BE-VP64, B3C2-RXR-LLL-BE-VP64, B3C2-RXR-L-DE-VP16, B3C2-RXR-LL-DE-VP16, B3C2-RXR-LLL-DE-VP16, B3C2-RXR-L-BE-VP16, B3C2 B-RXR-LL-BE-VP16, B3C2-RXR-LLL-BE-VP16;
Gene Switches Using RXR, E2C, and Repression Domains
E2C-RXR-L-DE-(KRAB-A)2, E2C-RXR-LL-DE-(KRAB-A)2, E2C-RXR-LLL-DE-(KRAB-A)2, B2C-RXR-L-BE-(KRAB-A)2, E2C-RXR-LL-BE-(KRAB-A)2, E2C-RXR-LLL-BE-(KRAB-A)2, E2C-RXR-L-DE-(KRAB-A)2, E2C-RXR-LL-DE-(KRAB-A)2, E2C-RXR-LLL-DE-(KRAB-A)2, E2C-RXR-L-BE-(KRAB-A)2, E2C-RXR-LL-BE-(KRAB-A)2, E2C-RXR-LLL-BE-(KRAB-A)2, E2C-RXR-L-DE-(SID)2, E2C-RXR-LL-DE-(SID)2, E2C-RXR-LLL-DE-(SID)2, E2C-RXR-L-BE-(SID)2, E2C-RXR-LL-BE-(Sm)2, E2C-RXR LLL-BE-(SID)2, E2C-RXR-L-DB-(SID)2, E2C-RXR-LL-DE-(SID)2, E2C-RXR-LLL-DE-(SID)2, B2C-RXR-L-BE-(SID)2, E2C-RXR-LL-BE-(SID)2, E2C-RXR-LLL-BE-(SID)2;
Gene Switches Using RXR. 2C7. and Repression Domains
2C7-RXR-L-DE-(KRAB-A)2,2C7-RXR-LL-DE-(KRAB-A)2, 2C7-RXR-LLL-DE-(KRAB-A)2,2C7-RXR-L-BE-(KRAB-A)2, 2C7-RXR-LL-BB-(KRAB-A)2,2C7-RXR-LLL-BE-(KRAB-A)2,2C7-RXR-L-DE-(KRAB-A)2,2C7-RXR-LL-DE-(KRAB-A)2,2C7-RXR-LLL-DE-(KRAB-A)2, 2C7-RXR-L-BE-(KRAB-A)2, 2C7-RXR-LL-BE-(KRAB-A)2, E2C-RXR-LLL-BE-(KRAB-A)2, 2C7-RXR-L-DE-(SID)2,2C7-RXR-LL-DB-(SID)2,2C7-RXR-LLL-DE-(SID)2,2C7-RXR-L-DE-(SID)2,2C7-RXR,-LL-BE-(SID)2,2C7-RXR-LLL,BE-(SID)2,2C7-RXR-L-DH-(SID)2,2C7-RXR-LL-DE-(SID)2,2C7-RXR-LLL-DE-(SID)2,2C7-RXR-L-BE-(SID)2,2C7-RXR-LL-BE-(SID)2, E2C-RXR-LLL-BE-(SID)2,n;
Gene Switches Using RXR, B3B, and Repression Domains
B3B-RXR-L-DE-(KRAB-A)2, B3B-RXR-LL-DB-(KRAB-A)2, B3B-RXR-LLL-DB-(KRAB-A)2, B3B 7-RXR-L-BE-(KRAB-A)2, B3B 7-RXR-LL-BE-(KRAB-A)2, B3B-RXR-LLL-BE-(KRAB-A)2, B3B-RXR-L-DE-(KRAB-A)2, B3B-RXR-LL-DB-(KRAB-A)2, B3B-RXR-LLL-DE-(KRAB-A)2, B3B-RXR-L-BE-(KRAB-A)2, B3B-RXR-LL-BB-(KRAB-A)2, B3B-RXR-LLL-BE-(KRAB-A)2, B3B-RXR-L-DE-(SID)2, B3B-RXR-LL-DE-(SID)2, B3B-RXR-LLL-DE-(SID)2, B3B 7-RXR-L-BE-(SID)2, B3B 7-RXR-LL-BE-(SID)2, B3B-RXR-LLL-BE-(SID)2, B3B-RXR-L-DE-(SID)2, B3B-RXR-LL-DB-(S7D)2, B3B-RXR-LLL-DE-(SID)2, B3B-RXR-L-BE-(SID)2, B3B-RXR-LL-BE-(SID)2, B3B-RXR-LLL-BE-(SID)2;
Gene Switches Using RXR, B3C2. and Repression Domains
B3C2-RXR-L-DE-(KRAB-A)2, B3C2-RXR-LL-DE-(KRAB-A)2, B3C2-RXR-LLL-DE-(KRAB-A)2, B3C2-RXR-L-BE-(KRAB-A)2, B3C2-RXR-LL-BE-(KRAB-A)2, B3C2-RXR-LLL-BE-(KRAB-A)2, B3C2-RXR-L-DB-(KRAB-A)2, B3C2-RXR-LL-DE(KRAB-A)2, B3C2-RXR-LLL-DE-(KRAB-A)2,B3C2-RXR-L-BE-(KRAB-A)2, B3C2 B-RXR-LL-BE-(KRAB-A)2, B3C2-RXR-LLL-BE-(KRAB-A)2, B3C2-RXR-L-DE-(SID)2, B3C2-RXR-LL-DE-(SID)2, B3C2-RXR-LLL-DE-(SID)2, B3C2-RXR-L-BE-(SID)2, B3C2-RXR-LL-BE-(SID)2, B3C2-RXR-LLL-BE-(SM)2, B3C2-RXR-L-DE-(SID)2, B3C2-RXR-LL-DE-(SID)2, B3C2-RXR-LLL-DE-(SID)2, B3C2,RXR-L-BE-(SID)2, B3C2 B-RXR-LL-BE-(SID)2, B3C2-RXR-LLL-BE-(SID)2;
Gene Switches Using PR, E2C, and Activation Domains
B2C-PR-L-PR-VP64, E2C-PR-LL-PR-VP64, E2C-PR-LLL-PR-VP64, B2C-PR-L-PR-VP64, E2C-PR-LL-PR-VP64, E2C-PR-LLL-PR-VP64, E2C-PR-L-PR-VP16, B2C-PR-LL-PR-VP16, E2C-PR-LLL-PR-VP16, E2C-PR-L-PR-VP16, E2C-PR-LL-PR-VP16, E2C-PR-LLL-PR-VP16;
Gene Switches Using PR, 2C7, and Activation Domains
2C7-PR-L-PR-VP64,2C7-PR-LL-PR-VP64,2C7-PR-LLL-PR-VP64, 2C7-PR-L-PR-VP64,2C7-PR-LL-PR-VP64,2C7-PR-LLL-PR-VP64,2C7-PR-L-PR-VP16, 2C7-PR-LL-PR-VP16, 2C7-PR-LLL-PR-VPI6, 2C7-PR-L-PR-VP16, 2C7-PR-LL-PR-VP16, E2C-PR-LLL-PR-VP16;
Gene Switches Using PR, B3B. and Activation Domains
B3B-PR-L-PR-VP64, B3B-PR-LL-PR-VP64, B3B-PR-LLL-PR-VP64, B3B 7-PR-L-PR-VP64, B3B 7-PR-LL-PR-VP64, B3B-PR-LLL-PR-VP64, B3B-PR-L-PR-VP16. B3B-PR-LL-PR-VP16, B3B-PR-LLL-PR-VP16, B3B-PR-L-PR-VP16, B3B-PR-LL-PR-VP16, B3B-PR-LLL-PR-VP16;
Gene Switches Using PR, B3C2, and Activation Domains
B3C2-PR-L-PR-VP64, B3C2-PR-LL-PR-VP64, B3C2-PR-LLL-PR-VP64, B3C2-PR-L-PR-VP64, B3C2-PR-LL-PR-VP64, B3C2-PR-LLL-PR-VP64, B3C2-PR-L-PR-VP16, B3C2-PR-LL-PR-VP16, B3C2-PR-LLL-PR-VP16, B3C2-PR-L-PR-VP16, B3C2 B-PR-LL-PR-VP16, B3C2-PR-LLL-PR-VP16;
Gene Switches Using PR. E2C, and Repression Domains
E2C-PR-L-PR-(KRAB-A)2, E2C-PR-LL-PR-(KRAB-A)2, E2C-PR-LLL-PR-(KRAB-A)2, E2C-PR-L-PR-(KRAB-A)2, E2C-PR-LL-PR-(KRAB-A)2, E2C-PR-LLL-PR-(KRAB-A)2, E2C-PR-L-PR-(KRAB-A)2, E2C-PR-LL-PR-(KRAB-A)2, E2C-PR-LLL-PR-(KRAB-A)2, E2C-FR-L-PR-(KRAB-A)2, E2C-PR-LL-PR-(KRAB-A)2, E2C-PR-LLL-PR-(KRAB A)2, E2C-PR-L-PR-(SID)2, E2C-PR-LL-PR-(SID)2, B2C-PR-LLL-PR-(SID)2, E2C-PR-L-PR-(SID)2, E2C-PR-LL-PR-(SID)2, E2C-PR-LLL-PR-(SID)2, E2C-PR-L-PR-(SID)2, E2C-PR-LL-PR-(SID)2, E2C-PR-LLL-PR-(SID)2, E2C-PR-L-PR-(SID)2, E2C-PR-LL-PR-(SID)2, E2C-PR-LLL-PR-(SID)2;
Gene Switches Using PR. 2C7. and Repression Domains
2C7-PR-L-PR-(KRAB-A)2,2C7-PR-LL-PR-(KRAB-A)2,2C7-PR-LLL-PR-(KRAB-A)2,2C7-PR-L-PR-(KRAB-A)2,2C7-PR-LL-PR-(KRAB-A)2,2C7-PR-LLL-PR-(KRAB-A)2,2C7-PR-L-PR-(KRAB-A)2,2C7-PR-PR-(KRAB-A)2,2C7-PR-LLL-PR-(KRAB-A)2,2C7-PR-L-PR-(KG2AB-A)2,2C7-PR-LL-PR-(KRAB-A)2, E2C-PR-LLL-PR-(KRAB-A)2, 2C7-PR-L-PR-(SID)2,2C7-PR-LL-PR-(SID)2,2C7-PR-LLL-PR-(SID)2,2C7-PR-L-PR-(SID)2,2C7-PR-LL-PR-(SID)2,2C7-PR-LLL-PR-(SID)2,2C7-PR-L-PR-(SID)2,2C7-PR-LL-PR-(SID)2, 2C7-PR-LLL-PR-(SID)2,2C7-FR-L-PR-(SID)2,2C7-PR-LL-PR-(SID)2, E2C-PR-LLL-PR-(SID)2,n;
Gene Switches Using PR. B3B. and Repression Domains
B3B-PR-L-PR-(KRAB-A)2, B3B-PR-LL-PR-(KRAB-A)2, B3B-PR-LLL-PR-(KRAB-A)2, B3B 7-PR-L-PR-(KRAB-A)2, B3B 7-PR-LL-PR-(KRAB-A)2, B3B-PR-LLL-PR-(KRAB-A)2, B3B-PR-L-PR-(KRAB-A)2, B3B-PR-LL-PR-(KRAB-A)2, B3B-PR-LLL-PR-(KRAB-A)2, B3B-PR-L-PR-(KRAB-A)2, B3B-PR-LL-PR-(KRAB-A)2, B3B-PR-LLL-PR-(KRAB-A)2, B3B-PR-L-PR-(SID)2, B3B-PR-LL-PR-(SID)2, B3B-PR-LLL-PR-(SID)2, B3B 7-PR-L-PR-(SID)2, B3B 7-PR-LL-PR-(SID)2, B3B-PR-LLL-PR-(SID)2, B3B-PR-L-PR-(SID)2, B3B-PR-LL-PR-(SID)2, B3B-PR-LLL-PR-(SID)2, B3B-PR-L-PR-(SID)2, B3B-PR-LL-PR-(SID)2, B3B-PR-LLL-PR-(SID)2;
Gene Switches Using PR, B3C2, and Repression Domains
B3C2-PR-L-PR-(KRAB-A)2, B3C2-PR-LL-PR-(KRAB-A)2, B3C2-PR-LLL-PR-(KRAB-A)2, B3C2-PR-L-PR-(KRAB-A)2, B3C2-PR-LL-PR-(KRAB-A)2, B3C2-PR-LLL-PR-(KRAB-A)2, B3C2-PR-L-PR-(KRAB-A)2, B3C2-PR-LL-PR-(KRAB-A)2, B3C2-PR-LLL-PR-(KRAB-A)2, B3C2-PR-L-PR-(KRAB-A)2, B3C2 B-PR-LL-PR-(KRAB-A)2, B3C2-PR-LLL-PR-(KRAB-A)2, B3C2-PR-L-PR-(SID)2, B3C2-PR-LL-PR-(SID)2, B3C2-PR-LLL-PR-(SID)2, B3C2-PR-L-PR-(SID)2, B3C2-PR-LL-PR-(SID)2, B3C2-PR-LLL-PR-(SID)2, B3C2-PR-L-PR-(SID)2, B3C2-PR-LL-PR-(SID)2, B3C2-PR-LLL-PR-(SID)2, B3C2-PR-L-PR-(SID)2, B3C2 B-PR-LL-PR-(SID)2, B3C2-PR-LLL-PR-(SID)2;
Gene Switches Using ER, E2C, and Activation Domains
E2C-ER-L-ER-VP64, E2C-ER-LL-ER-VP64, E2C-ER-LLL-ER-VP64, E2C-ER-L-ER-VP64, E2C-ER-LL-ER-VP64, E2C-BR-LLL-ER-VP64, B2C-ER-L-ER-VP16, E2C-ER-LL-ER-VP16, E2C-ER-LLL-ER-VP16, E2C-ER-L-ER-VP16, E2C-ER-LL-ER-VP16, E2C-ER-LLL-ER-VP16;
Gene Switches Using ER, 2C7, and Activation Domains
2C7-ER-L-ER-VP64, 2C7-ER-LL-ER-VP64, 2C7-ER-LLL-ER-VP64, 2C7-ER-L-ER-VP64, 2C7-ER-LL-ER-VP64, 2C7-ER-LLL-ER-VP64, 2C7 ER-L-ER-VP16,2C7-ER-LL-ER-VP16,2C7-ER-LLL-ER-VP16,2C7-ER-L-ER-VP16, 2C7-ER-LL-ER-VP16, E2C-ER-LLL-ER-VP16;
Gene Switches Using ER, B3B, and Activation Domains
B3B-ER-L-ER-VP64, B3B-ER-LL-ER-VP64, B3B-ER-LLL-ER-VP64, B3B 7-ER-L-ER-VP64, B3B 7-ER-LL-ER-VP64, B3B-ER-LLL-ER-VP64, B3B-ER-L-ER-VP16, B3B-ER-LLER-VP16, B3B-BR-LLL-ER-VP16, B3B-BR-L-ER-VP16, B3B-ER-LL-ER-VP16, B3B-ER-LLL-BR-VP16;
Gene Switches Using ER, B3C2, and Activation Domains
B3C2-ER-L-ER-VP64, B3C2-ER-LL-ER-VP64, B3C2-ER-LLL-ER-VP64, B3C2-ER-L-ER-VP64, B3C2-ER-LL-ER-VP64, B3C2-ER-LLL-ER-VP64, B3C2-ER-L-ER-VP16, B3C2-ER-LL-ER-VP16, B3C2-ER-LLL-ER-VP16, B3C2-ER-L-ER-VP16, B3C2 B-ER-LL-ER-VP16, B3C2-BR-LLL-ER-VP16;
Gene Switches Using ER, E2C, and Repression Domains
E2C-ER-L-ER-(KRAB-A)2, E2C-ER-LL-ER-(KRAB-A)2, E2C-ER-LLL-ER-(KRAB-A)2, E2C-ER-L-ER-(KRAB-A)2, E2C-ER-LL-ER-(KRAB-A)2, E2C-ER-LLL-ER-(KRAB-A)2, E2C-ER-L-ER-(KRAB-A)2, E2C-ER-LL-ER-(KRAB-A)2, B2C-ER-LLL-ER-(KRAB-A)2, E2C-ER-L-ER-(KRAB-A)2, E2C-ER-LL-ER-(KRAB-A)2, E2C-ER-LLL-ER-(KRAB-A)2, E2C-ER-L-ER-(SID)₂, E2C-ER-LL-ER-(SID)2, E2C-ER-LLL-ER-(SID)2, E2C-ER-L-ER-(SID)2, E2C-ER-LL-ER-(SID)2, E2C-ER-LLL-ER-(SID)2, E2C-ER-L-ER-(SID)2, E2C-ER-LL-BR-(SID)2, B2C-ER-LLL-ER-(SID)2, EZC-ER-L-ER-(SID)2, E2C-ER-LL-BR-(SID)2, E2C-ER-LLL-ER-(SID)2;
Gene Switches Using ER, 2C7, and Repression Domains
2C7-ER-L-ER-GULAB-A)2,2C7-ER-LL-ER-W,AB-A)2,2C7-ER-LLL-ER-(KRAB-A)2,2C7-ER-L-ER-(KRAB-A)2,2C7-ER-LL-ER-(KRAB-A)2, 2C7-BR-LLL-BR-(KRAB-A)2.2C7-ER-L-BR-(KRAB-A)2,2C7-ER-LL-BR-(KRAB-A)2,2C7-ER-LLL-ER-(KRAB-A)2,2C7-ER-L-ER-(KRAB-A)2,2C7-ER-LL-ER-(KRAB-A)2, E2C-ER-UL-ER-(KRAB-A)2, 2C7-ER-L-ER-(STD)2, 2C7-ER-LL-ER-(SID)2,2C7-ER-LLL-ER-(SID)2,2C7-ER-L-ER-(SID)2,2C7-ER-LL-ER-(SID)2,2C7-ER-LLL-ER-(SID)2,2C7-ER-L-ER-(SID)2,2C7-ER-LL-ER-(SID)2,2C7-BR-LLL-ER-(SID)2,2C7-ER-L-ER-(SID)2,2C7-ER-LL-ER-(SID)2, E2C-ER-LLL-ER-(SID)2,n;
Gene Switches Using ER, B3B, and Repression Domains
B3B-ER-L-ER-(KRAB-A)2, B3B-ER-LL-ER-(KRAB-A)2, B3B-ER-LLL-ER-(KRAB-A)2, B3B 7-ER-L-ER-(KRAB-A)2, B3B 7-ER-LL-ER-(KRAB-A)2, B3B-BR-LLL-ER-(KRAB-A)2, B3B-BR-L-BR-(KRAB-A)2, B3B-ER-LL-ER-(KRAB-A)2, B3B-ER-LLL-ER-(KRAB-A)2, B3B-ER-L-ER-(KRAB-A)2, B3B-ER-LL-ER-(KRAB-A)2, B3B-ER-LLL-ER-(KRAB-A)2, B3B-ER-L-ER-(SID)2, B3B-ER-LL-ER-(SID)2, B3B-ER-LLL-ER-(SID)2, B3B 7-ER-L-ER-(SID)2, B3B 7-ER-LL-ER-(SID)2, B3B-ER-LLL-ER-(SID)2, B3B-ER-L-ER-(SID)2, B3B-ER-LL-ER-(SID)2, B3B-ER-LLL-ER-(SID)2, B3B-ER-L-ER-(SID)2, B3B-ER-LL-ER-(SID)2, B3B-HR-LLL-ER-(SID)2;
Gene Switches Using ER, B3C2, and Repression Domains
B3C2-ER-L-ER-(KRAB-A)2, B3C2-ER-LL-ER-(KRAB-A)2, B3C2-ER-LLL-ER-(KRAB-A)2, B3C2-ER-L-ER-(KRAB-A)2, B3C2-BR-LL-ER-(KRAB-A)2, B3C2-ER-LLL-ER-(KRAB-A)2, B3C2-ER-L-ER-(KRAB-A)2, B3C2-ER-LL-ER-(KRAB-A)2, B3C2-ER-LLL-ER-(KRAB-A)2, B3C2-ER-L-ER-(KRAB-A)2, B3C2 B-ER-LL-ER-(KRAB-A)2, B3C2-ER-LLL-ER-(KRAB-A)2, B3C2-ER-L-ER-(SID)2, B3C2-ER-LL-ER-(SID)2, B3C2-ER-LLL-ER-(SID)2, B3C2-ER-L-ER-(SID)2, B3C2-ER-LL-ER-(SID)2, B3C2-ER-LLL-ER-(SID)2, B3C2-ER-L-ER-(SID)2, B3C2-ER-LL-BR-(SID)2, B3C2-ER-LLL-ER-(SID)2, B3C2-ER-L-ER-(SID)2, B3C2 B-ER-LL-ER-(SID)2, B3C2-ER-LLL-ER-(SID)2.

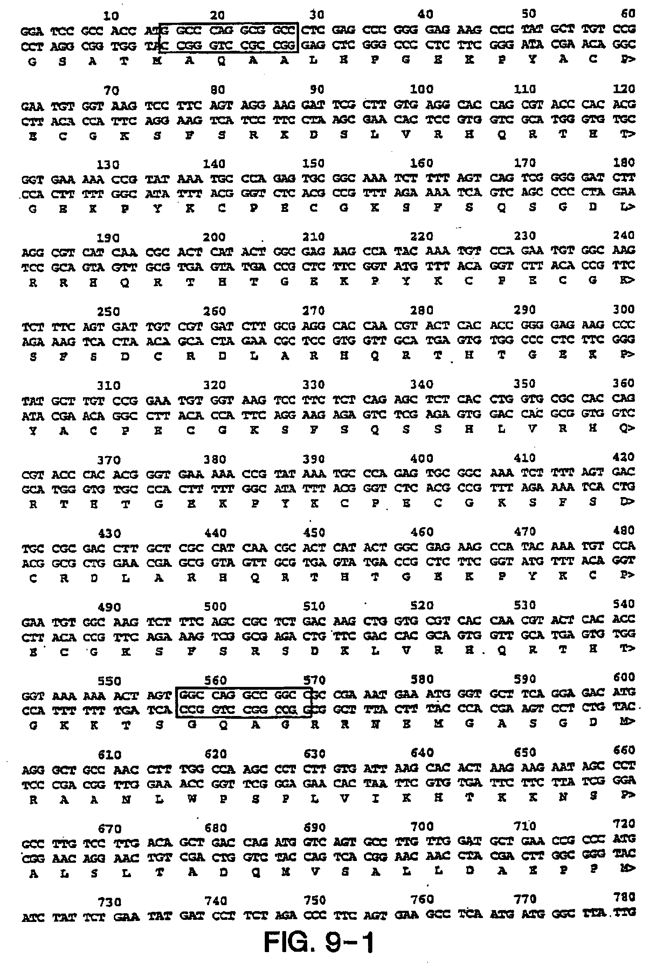

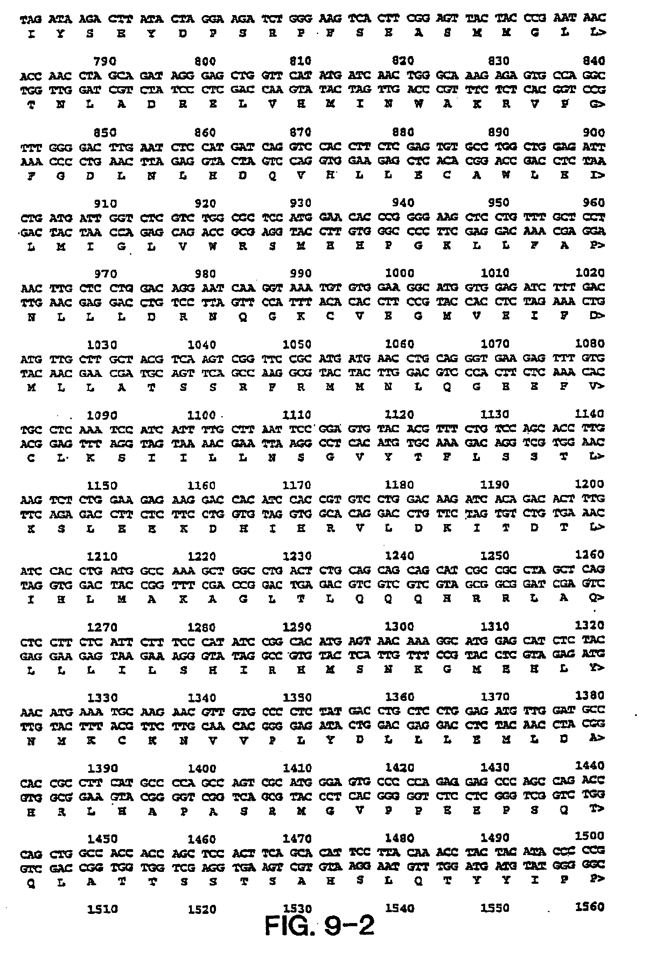

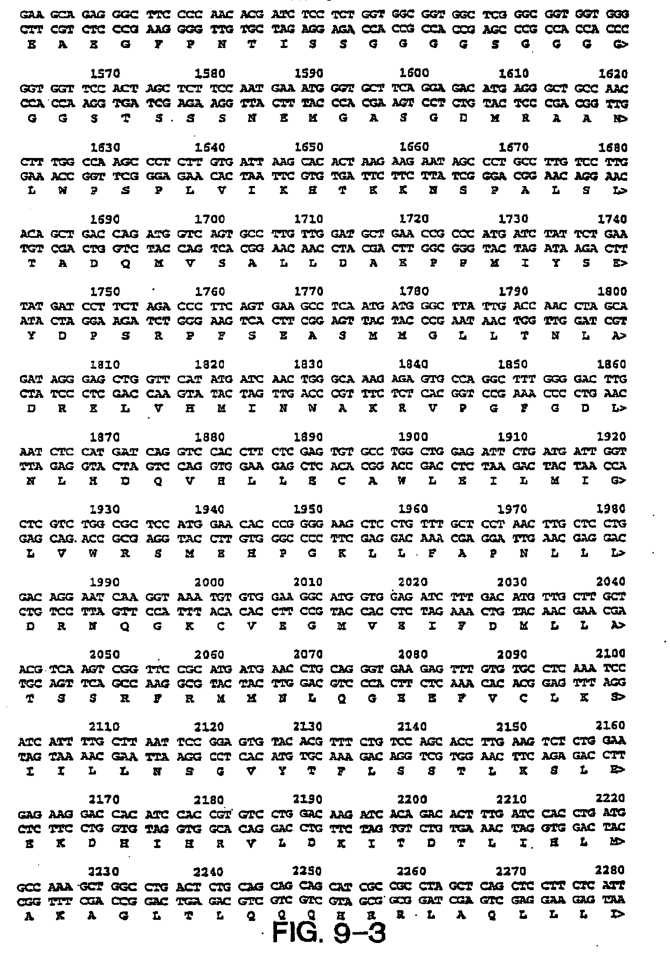

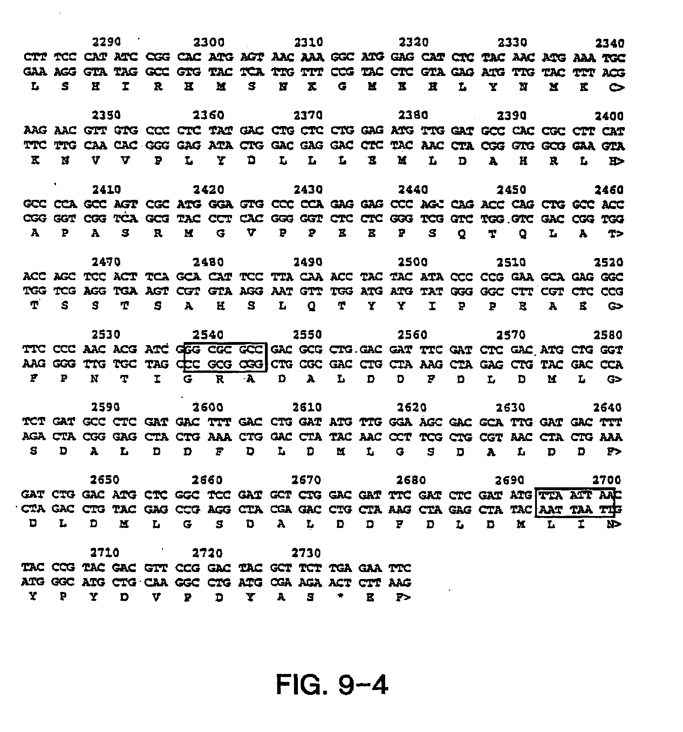

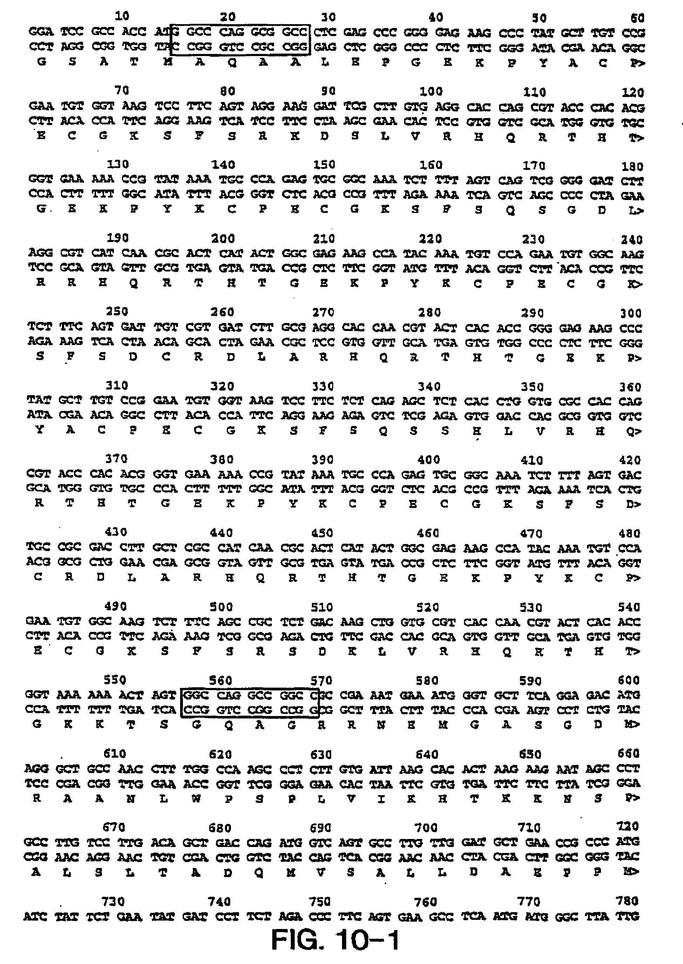

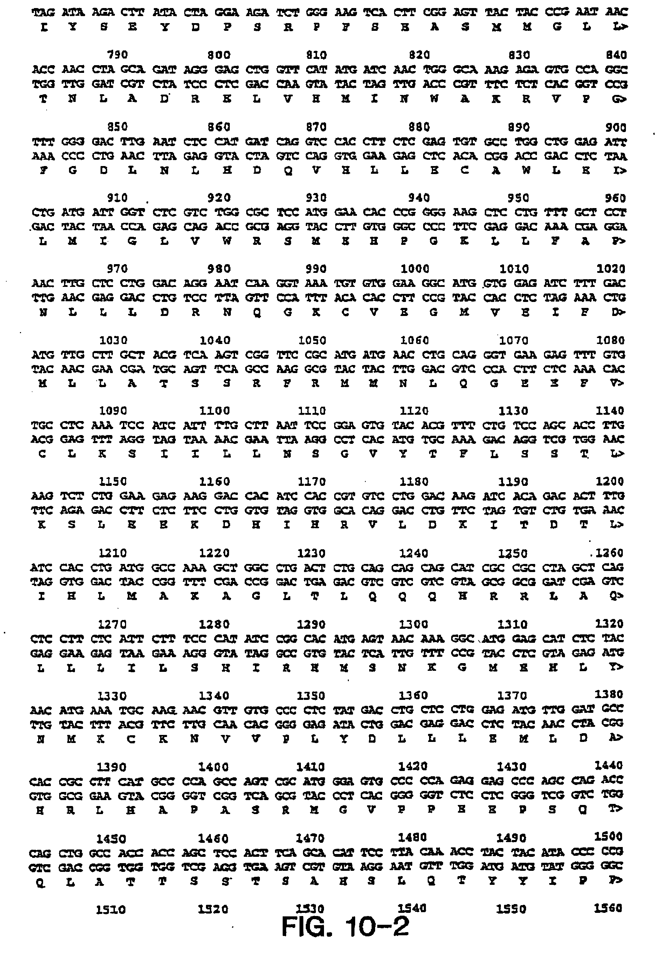

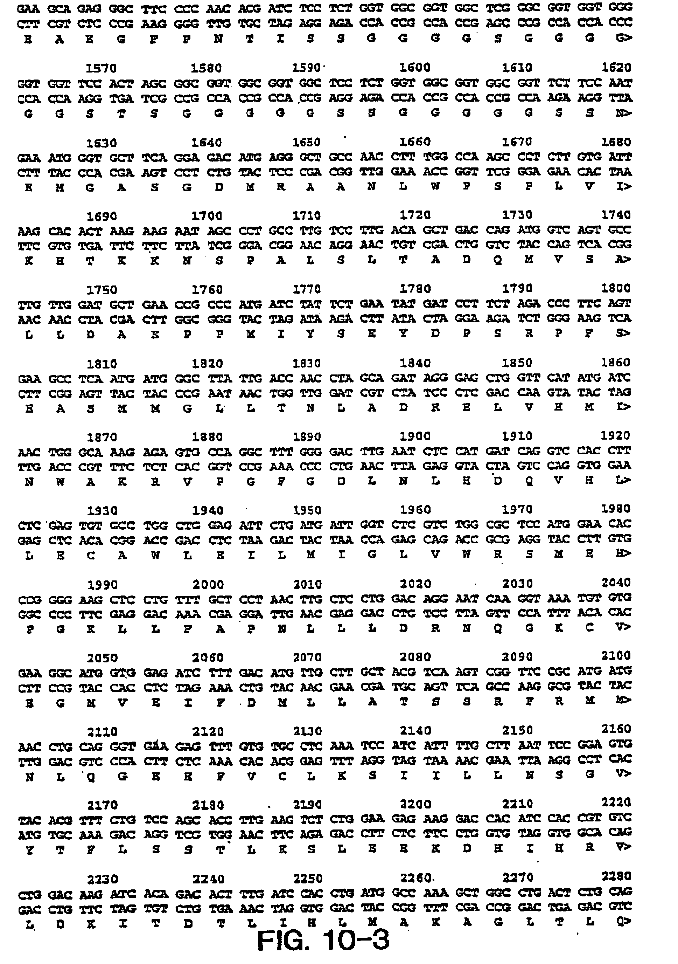

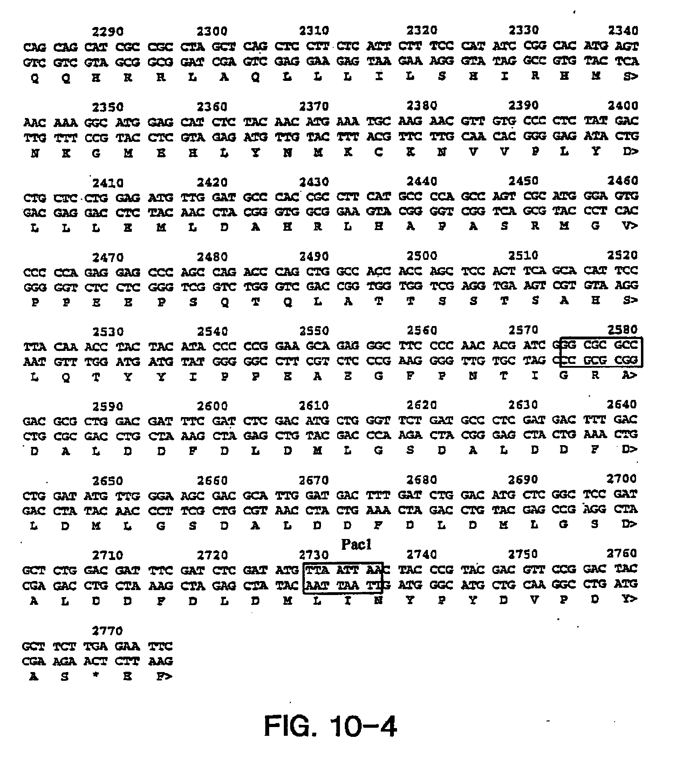

The nucleotide (SEQ ID NO: 39) and amino acid residue sequence (SEQ ID NO: 40) of polypeptide E2C-ER-L-ER-VP64 are shown in FIG. 9. The nucleotide (SEQ ID NO: 41) and amino acid residue sequence (SEQ ID NO: 42) of polypeptide E2C-ER-LL-ER-VP64 are shown in Fig.10.

### III. Polynucleotides, Expression Vectors and Host Cells

In a related aspect, the present invention provides polynucleotides that encode a polypeptide gene switch of this invention, expression vectors containing those polynucleotides, cells containing those polynucleotides and transformed cells containing those expression vectors. Vectors of primary utility for gene therapy include, but are not limited to human adenovirus vectors, adeno-associated vectors, murine or lenti virus derived retroviral vectors, or a variety of non-viral compositions including liposomes, polymers, and other DNA containing conjugates. Such vector systems can be used o deliver the gene switches either *in vitro* or *in vivo,* depending on the vector system. With adenovirus, for instance, vectors can be administered intravenously to transduce the liver and other organs, introduced directly into the lung, or into vascular compartments temporarily localized by ligation or other methods. Methods for constructing such vectors, and methods and uses for the described invention are known to those skilled in the field of gene therapy.

### IV. Methods of Regulating Nucleotide Function

The present invention further provides a process for regulating the expression of a desired nucleotide sequence such as a gene. In accordance with the process, the target nucleotide sequence is exposed to an effective amount of a gene switch and a ligand, wherein the nucleotide binding domain of the gene switch binds to a portion of the target nucleotide and wherein the ligand binds to at least one of the ligand binding domains of the gene switch. Exposure can occur *in vitro, in situ* or *in vivo.* The term "effective amount" means that amount that regulates transcription of a nucleotide (e.g. structural gene or translation ofRNA).

The term "regulating" refers to the suppression, enhancement, or induction of a function. For example, a polypeptide of the invention may modulate a promoter sequence by binding to a motif within the promoter, thereby enhancing or suppressing transcription of a gene operatively linked to the promoter nucleotide sequence. Alternatively, modulation may include inhibition of a gene where the polypeptide binds to the structural gene and blocks DNA dependent RNA polymerase from reading through the gene, thus inhibiting transcription of the gene. Alternatively, modulation may include inhibition of translation of a transcript.

The promoter region of a gene includes the regulatory elements that typically lie 5' to a structural gene. If a gene is to be activated, proteins known as transcription factors attach to the promoter region of the gene. This assembly resembles an "on switch" by enabling an enzyme to transcribe a second genetic segment from DNA to RNA. In most cases the resulting RNA molecule serves as a template for synthesis of a specific protein; sometimes RNA itself is the final product.

Regulation of gene expression or transcription can be accomplished both by exposing the target gene to a polypeptide switch of this invention or, preferably by transforming a cell that contains the target gene with an expression vector that contains a polynucleotide sequence that encodes a gene switch.

The Examples that follow illustrate particular embodiments of the present invention and are not limiting of the specification or claims in any way.

### EXAMPLE 1: General Methods

**Construction of zinc finger proteins.** For the construction of the B3 and N1 zinc finger proteins, DNA recognition helices from the Zif268 Finger 2 variants pmGAA, pmGAC, pmGGA, pmGGG, and pGTA were utilized [Segal, D. J., Dreier, B., Beerli, R. R., and Barbas, C. F., III (1999) Proc. Natl. Acad. Sci. USA 96, 2758-2763]. Three finger proteins binding the respective 9-bp target-sites were constructed by grafting the appropriate DNA recognition helices into the framework of the three finger protein Sp1C [Desjarlais, J. R., and Berg, J. M. (1993) Proc. Natl. Acad. Sci. USA 90, 2256-2260]; DNA fragments encoding the two 3 finger proteins were assembled from 6 overlapping oligonucleotides as described [Beerli, R. R., Segal, D. J., Dreier, B., and Barbas, C. F., III (1998) Proc. Natl. Acad. Sci. USA 95,14628-14633]. The three finger protein coding regions were then cloned into the bacterial expression vector pMal-CSS via Sfi1 digestion.

**Protein purification.** Moltose binding protein (MBP) fusion proteins were purified to >90% homogeneity using the Protein Fusion and Purification System (New England Biolabs), except that Zinc Buffer A (ZBA; 10 mM Tris, pH7.5/90 mM KC1, 1 mM MgCl₂, 90 µM ZnCl₂)/1% BSA /5 mM DTT) was used as the column buffer. Protein purity and concentration were determined from Coomassie blue-stained 15% SDS-PAGE gels by comparison to BSA standards.

**ELISA** analysis. In 96-well ELISA plates, 0.2 µg of streptavidin (Pierce) was applied to each well for 1 hour at 37°C, then washed twice with water. Biotinylated target oligonucleotide (0.025 µg) was applied in the same manner. ZBA/3% BSA was applied for blocking, but the wells were not washed after incubation. All subsequent incubations were at room temperature. Starting with 2 µg purified MBP fusion protein in the top wells, 2-fold serial dilutions were applied in 1x binding buffer (ZBA/1% BSA/5 mM DTT/0.12 µg/µl sheared herring sperm DNA). The samples were incubated 1 hour at room temperature, followed by 10 washes with water. Mouse anti-maltose binding protein mAb (Sigma) in ZBA/1% BSA was applied to the wells for 30 minutes, followed by 10 washes with water. Goat anti -mouse IgG mAb conjugated to alkaline phosphatase (Sigma) was applied to the wells for 30 minutes, followed by 10 washes with water. Alkaline phosphatase substrate (Sigma) was applied, and the OD₄₀₅ was quantitated with SOFTmax 2.35 (Molecular Devices).

**Gel mobility shift assays.** Target oligonucleotides were labeled at their 3' ends with [³²P] and gel purified. Eleven 3-fold serial dilutions of protein were incubated in 20 µl binding reactions (1x Binding Buffer/10% glycerol/≈1 pM target oligonucleotide) for three hours at room temperature, then resolved on a 5% polyacrlyamide gel in 0.5x TBE buffer. Quantitation of dried gels was performed using a Phosphorlmager and ImageQuant software (Molecular Dynamics), and the K_{D} was determined by Scatchard analysis.

**Reporter constructs for determining the optimal spacing and orientation of the two half-sites.** C7 dimer-TATA fragments were generated by PCR amplification with C7 dimer-TATA primers (5'-GAG GGT ACC GCG TGG GCG A₀₋₅ GCG TGG GCG AGT CGA CTC TAG AGG GTA TAT AAT GG-3' (SEQ ID NO: 1) for direct repeats; 5'-GAG GGT ACC GCG TGG GCG A₀₋₅ CGC CCA CGC AGT CGA CTC TAG AGG GTA TAT AAT GG-3' (SEQ ID NO: 2) for inverted repeats; 5'-GAG GGT ACC CGC CCA CGC A₀₋₅ GCG TGG GCG AGT CGA CTC TAG AGG GTA TAT AAT GG-3' (SEQ ID NO: 3) for everted repeats) and GLprimer2 (5'-CTT TAT GTT TTT GGC GTC TTC C-3' (SEQ ID NO: 4); Promega), using p17x4TATA-luc (gift from S. Y. Tsai) as a template. PCR products were cloned into pGL3-Basic (Promega) via digestion with the restriction endonucleases Kpn1 and Nco1.

**RU486- and Tamoxifen-inducible promoter constructs.** 10xC7-TATA, 10xB3-TATA, and 10xN1-TATA fragments were assembled from two pairs of complementary oligonucleotides each and cloned into Sac1-Xma1 linearized pGL3-Basic (Promega), upstream of the firefly luciferase coding region, creating the plasmids 10xC7-TATA-luc, 10xB3-TATA-luc, and 10xN1-TATA-luc. To generate the 10xN1-TATA-lacZ reporter construct, the lacZ coding region was excised from pβgal-Basic (Clontech) and used to replace the luciferase coding region of 10xN1-TATA-luc via Hind3-BamH1 digestion.

**Luciferase and β-gal reporter assays.** For all transfections, HeLa cells were plated in 24-well dishes and used at a confluency of 40-60%. For luciferase reporter assays, 175 ng reporter plasmid (promotor constructs in pGL3 or, as negative control, pGL3-Basic) and 25 ng effector plasmid (zinc finger-steroid receptor fusions in pcDNA3 or, as negative control, empty pcDNA3) were transfected using the Lipofectamine reagent (Gibco BRL). After approximately 24 h, expression was induced by the addition of 10nM RU486 (Biomol), 100 nM 4-OHT (Sigma), or 5 mM Ponasterone A (Invitrogen). Cell extracts were prepared approximately 48 hours after transfection and assayed for luciferase activity using the Promega luciferase assay reagent in a MicroLumat LB96P luminometer (EG&G Berthold, Gaithersburg, MD). For dual reporter assays, 85ng luciferase reporter plasmid, 85ng b-gal reporter plasmid, and 15ng of each of the two effector plasmids were transfected. b-gal activity was measured using the luminescent b-galactosidase detection kit II (Clontech).

**Zinc finger-steroid receptor fusion constructs with N-terminai effector domains.** The VP16 coding region was PCR amplified from pcDNA3/C7-VP16 using the primers VPNhe-F (5'-GAG GAG GAG GAG GCT AGC GCC ACC ATG GGG CGC GCC GGC GCT CCC CCG ACC GAT GTC AGC CTG-3') (SEQ ID NO: 5), and VPHind-B (5'-GAG GAG GAG GAG AAG CTT GTT AAT TAA ACC GTA CTC GTC AAT TCC AAG GGC ATC G-3') (SEQ ID NO: 6) or VPNLSHind-B (5'-GAG GAG GAG GAG AAG CTT AAC TTT GCG TTT CTT TTT CGG GTT AAT TAA ACC GTA CTC GTC AAT TCC AAG GGC ATC G-3') (SEQ ID NO: 7). The C7 coding region was amplified from the same plasmid, using the primers C7Hind-F (5'-GAG GAG GAG GAG AAG CTT GGG GCC ACG GCG GCC CTC GAG CCC TAT GC-3') (SEQ ID NO: 8), and C7Bam-B (5'-GAG GAG GGA TCC CCC TGG CCG GCC TGG CCA CTA GTT CTA GAG TC-3') (SEQ ID NO: 9) or C7NLSBam-B (5'-GAG GAG GGA TCC CCA ACT TTG CGT TTC TTT TTC GGC TGG CCG GCC TGG CCA CTA GTT CTA GAG TC-3') (SEQ ID NO: 10). The human PR truncated LBD (aa645-914) was amplified from PAPCMVGL914VPc'-SV [Wang, Y., Xu, J., Pierson, T., O'Malley, B. W., and Tsai, S. Y. (1997) Gene Therapy 4, 432-441] using the primers PRBam-F (5'-GAG GAG GAG GAG GGA TCC AGT CAG AGT TGT GAG AGC ACT GGA TGC TG-3') (SEQ ID NO: 11) and PREco-B (5'-GAG GAG GAA TTC TCA AGC AAT AAC TTC AGA CAT CAT TTC TGG AAA TTC-3') (SEQ ID NO: 12). The VP16-C7-PR, VP16-NLS-C7-PR, and VP16-C7-NLS-PR coding regions were then assembled in pcDNA3.1(+)Zeo (Invitrogen) using the Nhe1, Hind3, BamH1, and EcoR1 restriction sites incorporated in the PCR primers. In the resulting constructs, the C7 coding regions were flanked by two Sfi1 sites, and the VP16 coding regions by Asc 1 and Pac 1 sites. These restriction sites were introduced to facilitate the exchange ofDBDs and effector domains, respectively.

To generate the VP16-C7-ER, VP16-NLS-C7-ER, and VP16-C7-NLS-ER constructs, the point-mutated murine ER LBD coding region (aa281-599, G525R) was excised from pBabe/Myc-ER [Littlewood, T. D., Hancock, D. C., Danielian, P. S., Parker, M. G., and Evan, G. L (1995) Nutl. Acids Res. 23, 1686-1690], and used to replace the PR LBD coding region via BamH1-EcoR1 restriction digestion.

To generate fusion constructs with B3 or N1 DBDs, C7 was replaced by the B3 or N1 coding regions via Sfi1 digestion. Fusion constructs containing a VP64 effector domain were produced by replacing VP16 by the VP64 coding region via Asc1-Pac1 digestion.

**Zinc finger-steroid receptor fusion constructs with C-terminal effector domains.** The truncated human PR LBD was amplified from PAPCMVGL914VPc'-SV [Wang, Y., Xu, J., Pierson, T., O'Mailey, B. W., and Tsai, S. Y. (1997) Gene Therapy 4, 432-441] using the primers PRFse-F (5'- GAG GAG GAG GAG GAG GGC CGG CCG CGT CGA CCA GGT CAG AGT TGT GAG AGC ACT GGA TGC-3') (SEQ ID NO:13) and PRAsc-B (5'- GAG GAG GAG GAG GAG GGC GCG CCC CGT CGA CCC AGC AAT AAC TTC AGA CAT CAT TTC TGG-3') (SEQ ID NO: 14). The point-mutabed mouse ER LBD was amplified from pBabe/Myc-ER [Littlewood, T. D., Hancock, D. C., Danielian, P. S., Parker, M. G., and Evan, G. I. (1995) Nucl. Acids Res. 23, 1686-1690] using the primers ERFse-F (5'- GAG GAG GAG GAG GAG GGC CGG CCG CCG AAA TGA AAT GGG TGC TTC AGG AGA C-3') (SEQ ID NO: 15) and ERAsc-B (5'- GAG GAG GAG GAG GAG GGC GCG CCC GAT CGT GTT GGG GAA GCC CTC TGC TTC-3') (SEQ ID NO:16). The resulting PCR products were then inserted into pcDNA3/E2C-VP16 [Beerli, R. R., Segal, D. J., Dreier, B., and Barbas, C. F., III (1998) Proc. Natl. Acad. Sci. USA 95, 14628-14633], in between the E2C and VP16 coding regions, via digestion with the restriction endonucleases Fse1 and Asc1.

To generate fusion constructs with B3 or N1 DBDs, E2C was replaced by the B3 or N1 coding regions via Sfi1 digestion. Fusion constructs containing a VP64 effector domain were produced by replacing VP16 by the VP64 coding region via Ascl-Pac1 digestion.

**Heterodimeric switch constructs.** For construction of the E2C-ER fusion, the point mutated mouse ER LBD was amplified from pBabe/Myc-ER [Littlewood, .T. D., Hancock, D. C., Danielian, P. S., Parker, M. G., and Evan, G. I. (1995) Nucl. Acids Res. 23, 1686-1690] using the primers ERFse-F and ERPac-B (5'-GAG GAG GAG GAG GAG TTA ATT AAG ATC GTG TTG GGG AAG CCC TCT GCT TC-3') (SEQ ID NO: 17). The PCR product was then inserted into the construct pcDNA3/E2C-VP64, replacing the VP64 coding region, via Fse1-Pac1 digestion. To generate the ER-VP64 fusion, the ER LBD was amplified using the primers ERATGBam-F (5'-GAG GAG GAG GAG GGA TCC GCC ACC ATG CGA AAT GAA ATG GGT GCT TCA GGA GAC-3') (SEQ ID NO: 18) and ERAsc-B. The PCR product was then inserted into pcDNA3/E2C-VP64, [Beerli, R. R., Segal, D. J., Dreier, B., and Barbas, C. F., III (1998) Proc. Natl. Acad. Sci. USA 95, 14628-14633] replacing the E2C coding region, via BamHl-Ascl digestion.

**Single-chain switch constructs.** For construction of single-chain fusions with two ER LBDs, the point-mutated mouse ER LBD was amplified from pBabe/Myc-ER [Littlewood, T. D., Hancock, D. C., Danielian, P. S., Parker, M. G., and Evan, G. I. (1995) Nucl. Acids Res. 23, 1686-1690] either using the primers ERFse-F and ERSpe1-B (5'-GAG GAG GAG GAG GAG GAG ACT AGT GGA ACC ACC CCC ACC ACC GCC CGA GCC ACC GCC ACC AGA GGA GAT CGT GTT GGG GAA GCC CTC TGC-3') (SEQ ID NO: 19), or using the primers ERNhel-F1 (for 18aa linker construct; 5'-GAG GAG GAG GAG GAG GAG GCT AGC GGC GGT GGC GGT GGC TCC TCT GGT GGC GGT GGC GGT TCT TCC AAT GAA ATG GGT GCT TCA GGA GAC-3') (SEQ ID NO: 20) or ERNheI-FZ (for 30aa linker construct; 5'- GAG GAG GAG GAG GAG GAG GCT AGC TCT TCC AAT GAA ATG GGT GCT TCA GGA GAC -3') (SEQ ID NO: 21), and ERAsc-B. The PCR products were then digested with, respectively, Fse1 and Spe1, or Nhe1 and Asc1, and inserted into Fse1-Asc1 linearized pcDNA3/E2C-VP64 [Beerli, R. R., Segal, D. J., Dreier, B., and Barbas, C. F., III (1998) Proc. Natl. Acad. Sci. USA 95, 14628-14633].

For construction of RXR-EcR single-chain fusions, the ligand binding domain of the human retinoid X receptor (hRXRα, aa373-654) was PCR amplified from pVgRXR (Invitrogen) using the primers RXRFse-F (5'-GAG GAG GAG GGC CGG CCG GGA AGC CGT GCA GGA GGA GCG GC-3') (SEQ ID NO: 22) and RXRSpe-B (5'-GAG GAG GAG GAG GAG ACT AGT GGA ACC ACC CCC ACC ACC GCC CGA GCC ACC GCC ACC AGA GGA AGT CAT TTG GTG CGG CGC CTC CAG C-3') (SEQ ID NO: 23). The ligand binding domain of the ecdysone receptor (EcR, aa202-462, *drosophila melanogaster*) was PCR amplified from pVgRXR using the primers EcRNhe-F1 (for 18aa linker construct; 5'-GAG GAG GAG GAG GCT AGC TCT TCC GGT GGC GGC CAA GAC TTT GTT AAG AAG G-3') (SEQ ID NO: 24), or EcRNhe-F2 (for 30aa linker construct; 5'-GAG GAG GAG GAG GCT AGC GGC GGT GGC GGT GGC TCC TCT GGT GGC GGT GGC GGT TCT TCC GGT GGC GGC CAA GAC TTT GTT AAG AAG G-3') (SEQ ID NO: 25), and EcRAsc-B (5'-GAG GAG GAG GGC GCG CCC GGC ATG AAC GTC CCA GAT CTC CTC GAG-3') (SEQ ID NO: 26). The PCR products were then digested with, respectively, Fse1 and Spe1, or Nhe1 and Asc1, and inserted into Fset-Asc 1 linearized pcDNA3/E2C-VP64 [Beerli, R. R., Segal, D. J., Dreier, B., and Barbas, C. F., III (1998) Proc. Natl. Acad. Sci. USA95, 14628-14633]. DNA binding domains were exchanged via Sfi1 digestion, effector domains via Asc1-Pacl digestion.

To generate the 36aa linker, E2C-RLLE-VP64 fusion construct, the RXR LBD was PCR amplified from pcDNA3/E2C-RE-VP64 using the primers RXRFse-F and RXRSpeLL-B (5'-GAG GAG GAG GAG GAG ACT AGT AGA GCC ACC GCC CCC TTC AGA ACC GCC CGA GCC ACC GCC ACC AGA GG-3') (SEQ ID NO: 27). The EcR LBD was amplified from the same plasmid, using the primers EcRNheLL-F (5'-GAG GAG GAG GAG GCT AGC GGG GGT TCG GAG GGT GGC GGG TCT GAG GGT GGG GGT GGT TCC ACT AGC TCT TCC-3') (SEQ ID NO: 28) and EcRAsc-B. The PCR products were inserted into pcDNA3/E2C-VP64 as described above.

### EXAMPLE 2: Gene Switches

**Generation of hormone-regulated zinc finger-steroid receptor fusion proteins.** Previous studies have shown the potential of engineered C2-H2 zinc finger proteins for the regulation of target gene expression [. Liu, Q., Segal, D. J., Ghiara, J. B., and Barbas, C. F., III (1997) Proc. Natl. Acad. Sci. USA 94, 5525-5530; Kim, J. S., and Pabo, C. O. (1997) J Biol Chem 272, 29795-29800;. Beerli, R. R., Segal, D. J., Dreier, B., and Barbas, C. F., III (1998) Proc. Natl. Acad. Sci. USA 95, 14628-14633; Beerli, R. R., Dreier, B., and Barbas, C. F., III (2000) Proc. Natl. Acad. Sci. USA 97, 1495-1500]. However, to fully realize the potential of engineered zinc finger proteins, it is desirable that their otherwise constitutive DNA binding activity be rendered ligand-dependent. The ligand binding domains (LBDs) of the human progesterone receptor (hPR) and the murine estrogen receptor (mER) have previously been used for the regulation of heterologous proteins, after having been modified to lack binding to the natural hormones while retaining binding to synthetic antagonists [Littlewood, T. D., Hancock, D. C., Danielian, P. S., Parker, M. G., and Evan, G. I. (1995) Nucl. Acids Res. 23, 1686-1690; Wang, Y., Xu, J., Pierson, T., O'Malley, B. W., and Tsai, S. Y. (1997) Gene Therapy 4, 432-441]. Thus, the Zif268 variant C7 [Wu, H., Yang, W.-P., and Barbas, C. F., III (1995) Proc. Natl. Acad. Sci. USA 92, 344-348] was fused to a transcriptional activation domain plus the LBD of either of the two nuclear hormone receptors. The VP64-C7-PR fusion protein contains an N-terminal VP64 activation domain [Beerli, R. R., Segal, D. J., Dreier, B., and Barbas, C. F., III (1998) Proc. Natl. Acad. Sci. USA 95, 14628-14633], and a C-terminal hPR LBD (aa645-914) lacking amino acids 915-933, responsive to the progesterone-antagonist RU486/Mifepristone but not to progesterone [Wang, Y., Xu, J., Pierson, T., O'Malley, B. W., and Tsai, S. Y. (1997) Gene Therapy 4,432-441]. The VP64-C7-ER fusion protein contains a C-terminal mER LBD (aa282-599) with a single amino acid substitution (G525R), and is responsive to the estrogen antagonist 4-hydroxy-tamoxifen (4-OHT) but not to estrogen [Littlewood, T.D., Hancock, D. C., Danielian, P. S., Parker, M. G., and Evan, G. I. (1995) Nucl. Acids Res. 23, 1686-1690].

**Determination of the optimal response element for zinc finger-steroid receptor fusion proteins.** Naturally occurring steroid receptors bind DNA as dimers and typically recognize response elements consisting of palindromic sequences [Evans, R. M. (1988) Science 240, 889-895; Carson-Jurica, M. A., Schrader, W. T., and O'Malley, W. (1990) Endocrine Reviews 11, 201-220]. Moreover, it was demonstrated that in some cases also direct repeats can serve as binding sites for receptor dimers [Aumais, J. P., Lee, H. S., DeGannes, C., Horsford, J., and White, J. H. (1996) J. Biol. Chem. 271, 12568-12577]. Given this obvious flexibility in DNA recognition by naturally occurring receptor dimers, the optimal structure of a response element for an artificial, zinc finger based transcriptional switch was not known. However, to develop an efficient, hormone-inducible system for the regulation of target gene expression, a detailed knowledge of the binding site architecture is required.

To determine the optimal orientation and spacing of the two half-sites of a response element for a zinc finger-LBD fusion protein, a series of reporter plasmids was constructed. Each contains two C7 binding sites upstream of a TATA box and a firefly luciferase coding region. The two C7 binding sites were introduced in different orientations (direct, inverted, or everted repeat) and with various spacings (no spacing or 1 to 5 bp spacing). Plasmids directing expression of VP64-C-PR or VP64-C7-ER fusion constructs were then co-txansfected with the various reporter plasmids and assayed for hormone-induced luciferase expression. Significantly, each of the C7 dimer binding sites was able to act as a response element for both PR and ER based proteins, albeit at variing efficiency. In contrast, a reporter plasmid with a single C7 binding site was not activated, indicating that hormone-induced activation of transcription was mediated by dimers.

Optimal spacing depended on the orientation of the two half-sites. In the case of the PR fusion protein, optimal spacing seemed to be at 2-3 bp for inverted repeats and 3 bp for everted repeats. Response elements consisting of direct repeats had no single optimal spacing; the best response was obtained with 4-5 bp, or no spacing at all. For the ER fusion protein, optimal spacing was at 3-4 bp for direct repeats, 1-2 bp for inverted repeats, and 3 bp for everted repeats . It should be noted that there were significant variations in the basal, i.e. ligand-independent activity of PR and ER fusion proteins, depending on the response element tested. Most notably, increasing the spacing of direct repeats from 3 to 4 bp led to a 1.9-fold higher basal activity of VP64-C7-PR, and even a 3.7-fold increase in the case of VP64-C7-ER. High basal activity is extremely undesirable for an inducible promoter system, where tight control over the expression levels of a particular gene of interest is often required, especially if the gene product is toxic. Thus, in choosing appropriate response elements, particular attention must be paid not only to hormone inducibility but also to its basal activity in the presence of the regulatory protein. The response element consisting of direct repeats with a spacing of three nucleotides was considered to be a good choice for use in a hormone-inducible artificial promoter, since it was compatible with both PR and ER fusion proteins. Significantly, its basal acticity in the presence of either PR or ER fusion proteins was among the lowest of all response elements tested. Furthermore, good hormone induced activation of transcription was observed with both VP64-C7-PR (3.9-fold) and VP64-C7-ER (9.5-fold).

**Generation of novel DNA binding domains.** While the use of the C7 DNA binding domain was well suited for the preliminary studies described above, it may not be a good choice for incorporation into an inducible transcriptional regulator. The C7 protein is a variant of the mouse transcription factor Zif268 [Pavletich, N. P., and Pabo, C. O. (1991) Science 252, 8U9-817], with increased affinity but unchanged specificity [Wu, H., Yang, W.-P., and Barbas, C. F., III (1995) Proc. Natl. Acad Sci. USA 92, 344-348]. We reasoned that the use of alternate DNA binding domains would minumize potential pleiotropic effects of the chimeric regulators. Previously, we described a strategy for the rapid assembly of zinc finger proteins from a family of predefined zinc finger domains specific for each of the sixteen 5'-GNN-3' DNA triplets [Beerli, R. R., Segal, D. J., Dreier, B., and Barbas, C. F., III (1998) Proc. Natl. Acad. Sci. USA 95, 14628-14633; Segal, D. J., Dreier, B., Beerli, R. R., and Barbas, C. F., III (1999) Proc. Natl. Acad. Sci. USA 96, 2758-2763]. Three finger proteins binding any desired 5'-(GNN)₃-3' sequence can be rapidly prepared by grafting the amino acid residues involved in base-specific DNA recognition into the framework of the consensus three finger protein Sp1C [Desjarlais, J. R., and Berg, J. M. (1993) Proc. Natl. Acad. Sci. USA 90, 2256-2260]. To date, well over 100 three finger proteins have been produced in our laboratory. Two of these, B3 and N1, were chosen to be used in inducible transcriptional regulators (Figure 1A). The B3 and N 1 proteins are designed to bind the sequences 5'-GGA GGG GAC-3' or 5'-GGG GTA GAA 3', respectively. To verify their DNA binding specificity, these proteins were purified as MBP-fusions and tested by ELISA analysis using an arbitrary selection of oligonucleotides containing 5'-(GNN)₃-3'sequences (Fig. 1B). Significantly, both proteins recognized their target sequence and showed no crossreactivity to any of the other 5'-(GNN)₃-3' sequences tested. However, as judged by ELISA, binding of N1 was much weaker than binding of B3. Therefore, affinities were determined by electrophoretic mobility-shift analysis. The B3 protein bound its target sequence with a K_{D} value of 15nM, similar to the K_{D} values we previously reported for other three finger proteins [Beerli, R. R., Segal, D. J., Dreier, B., and Barbas, C. F., III (1998) Proc. Natl. Acad Sci. USA 95, 14628-14633]. In contrast, N1 affinity for its target was dramatically lower and we estimate its K_{D} value to be in the range of 5-10 µM. The fact that the two proteins had very different affinities for their respective target sequences was considered positive, since it allows to investigate the influence of affinity on the functionality of an inducible expression system.

**RU486- and 4-OHT-inducible systems for the control of gene expression.** To allow for a comparative analysis, a series of RU486- or 4-OHT-inducible transcriptional regulators were constructed containing either the B3 or the N1 DNA binding domain. The role of placement of the activation domain was investigated, by fusing it either to the N- or the C-termimis of the protein. Two different activation domains were compared: the *herpes simplex* virus VP16 transactivation domain [Sadowski, I., Ma, J., Triezenberg, S., and Ptasbne, M. (1988) Nature 335, 563-564], and the synthetic VP64 activation domain, which consists of 4 tandem repeats of VP16's minimal activation domain [Beerli, R. R., Segal, D. J., Dreier, B., and Barbas, C. P., III (1998) Proc. Natl. Acad. Sci. USA 95, 14628-14633].

Synthetic promoters were constructed based on the B3 and N1 DNA target sequences, and the optimal response element structure defined above. The 10xB3-TATA-luc and 10xN1-TATA-luc plasmids each contain five response elements, consisting of direct repeats spaced by three nucleotides, upstream of a TATA box and a firefly luciferase coding region. The response elements are separated from each other by six nucleotides, which should allow the concomitant binding of five dimers and thus maximize the promoter activity. The activity of the various fusion constructs was assessed by transient cotransfection studies with the cognate TATA reporter plasmids in HeLa cells (Table 1).

**Table 1**

| | **LBD=PR** | | **LBD=ER** | |
|---|---|---|---|---|
| | **exp. 1** | **exp.2** | **exp. 1** | **exp.2** |
| **VP16-B3-LBD** | **34x** | **36x** | **37x** | **26x** |
| **VP64-B3-LBD** | **37x** | **24x** | **26x** | **27x** |
| **B3-LBD-VP16** | **115x** | **116x** | **47x** | **58x** |
| **B3-LBD-VP64** | **110x** | **85x** | **62x** | **99x** |
| **VP16-N1-LBD** | **188x** | **159x** | **101x** | **39x** |
| **VP64-N1-LBD** | **206x** | **390x** | **49x** | **58x** |
| **N1-LBD-VP16** | **282x** | **203x** | **24x** | **30x** |
| **N1-LBD-VP64** | **151x** | **129x** | **1319x** | **464x** |

In general, the ER fusion proteins turned out to be the stronger transactivators, and 4-OHT-induced luciferase activity was usually 3 to 6 times higher than RU486-induced luciferase activity mediated by PR fusion proteins. However, since the basal, i. e. ligand independent, activity of ER chimeras was often somewhat higher, their hormone-induced fold-stimulation was not generally better. Hormone-dependent gene activation in excess of 2 orders of magnitude was commonly observed with both PR and ER fusion proteins, values that are significantly better than what was previously reported for the Gal4-PR fusion protein GLVPc' [Wang, Y., Xu, J., Pierson, T., O'Malley, B. W., and Tsai, S. Y. (1997) Gene Therapy 4, 432-441].

The placement of the activation domain had a significant influence on the activity of the chimeric regulators. However, favored placement was dependent on the nature of the activation domain. Whereas the VP16 domain yielded the more potent activators when placed at the C-terminus, the VP64 was more active at the N-terminus. Accordingly, direct comparisons showed that an N-terminal VP64 was more potent than a N-terminal VP16 domain, and a C-terminal VP16 was more potent than a C-terminal VP64 domain. The nature and placement of the activation domain was also found to have an influence on the basal activity of the chimeric regulators. In particular, a relatively high basal activity was observed in the case of regulators with N-terminal VP64 domain.

The nature of the DNA binding domain had a major influence on the extent of ligand-dependence of the chimeras. Use of the N1 protein as DNA binding domain led to more tightly regulated fusion constructs with significantly better fold-stimulation of promoter activities than the use of B3, likely due to the dramatic affinity differences between N1 and B3. In particular, the N1-ER-VP64 regulator had no significant basal activity and was capable of mediating a 464- to 1319-fold 4-OHT-induced activation of the 10xN1-TATA minimal promoter (Table 1). The extent of ligand-induced activation of gene expression over a range of 3 orders of magnitude is particularly remarkable, since it has thus far only been reported for the tetracycline controlled system of gene regulation [Gossen, M., and Bujard, H. (1992) Proc. Natl. Acad. Sci. USA 89, 5547-5551; Gossen, M., Freundlieb, S., Bender, G., Müller, G., Hillen, W., and Bujard, H.(1995) Science 268, 1766-1769].

**Concomitant regulation of multiple promoters.** Zinc finger technology has made a large repertoire of DNA binding specificities available for use in protein engineering [Beerli, R R., Segal, D. J., Dreier, B., and Barbas, C. F., III (1998) Proc. Natl. Acad. Sci. USA 95, 14628-14633; Segal, D.J., Dreier, B., Beerli, R. R., and Barbas, C. F., III (1999) Proc. Natl. Acad. Sci. USA 96, 2758-2763; Beerli, R. R., Dreier, B., and Barbas, C. F., III (2000) Proc. Natl. Acad Sci. USA 97, 1495-1500]. The availability of different steroid hormone receptor-derived regulatory domains [Littlewood, T. D., Hancock, D. C., Danielian, P. S., Parker, M. G., and Evan, G. I. (1995) Nucl. Acids Res. 23,1686-1690; Wang, Y., Xu, J., Pierson, T., O'Malley, B. W., and Tsai, S. Y. (1997) Gene Therapy 4, 432-441], and the ability to redirect chimeric regulators to virtually any desired target sequence should make it possible to independently regulate the expression of multiple genes at the same time. To examine this possibility, a reporter plasmid was constructed directing expression of β-galactosidase (β-gal) under the control of the 10xN1-TATA minimal promoter. The chimeric regulators B3-PR-VP16 and N1-ER-VP64 were then transiently expressed in HeLa cells along with the 10xB3-TATA-luc and 10xN1-TATA-β-gal reporter plasmids. The transfected cells were treated with either RU486 or 4-OHT and the luciferase and β-gal activities were monitored. Significantly, RU486 induced expression of luciferase while having no effect on β-gal reporter gene activity. 4-OHT, on the other hand, did not affect luciferase expression but efficiently activated β-gal expression. These results demonstrate that the two regulator/promoter combinations act independently from one another, and that multiple genes can efficiently and independently regulated by the selective addition of the desired hormone.

**Development of a monomeric hormone-dependent gene-switch.** The ability to engineer DNA binding proteins with desired specificities makes it possible to generate artificial transcription factors capable of imposing dominant regulatory effects on endogenous genes [Beerli, R. R., Dreier, B., and Barbas, C. F., III (2000) Proc. Natl. Acad. Sci. USA 97, 1495-1500]. For many applications of this technology it may be desirable that the effect on endogenous gene expression is reversible. The use of steroid hormone receptor LBDs has the potential to render regulation of endogenous gene expression reversible. However, one major drawback is the fact that steroid hormone receptors, as well as the chimeric regulators described herein, bind DNA as dimers. Thus, when the fusion protein C7-ER-VP64 was transiently expressed in HeLa cells it was unable to regulate a reporter construct carrying a single C7 binding site, while it readily regulated a reporter that had two C7 binding sites and therefore accommodated binding of a dimer (Fig. 2B). An additional problem was encountered when the C7 DBD was replaced by E2C, which contains six zinc finger domains and recognizes the 18-bp sequence 5'-GGG GCC GGA GCC GCA GTG-3' (SEQ ID NO; 29) in the 5'-UTR of the proto-oncogene *c-erbB-2* [Yamamoto, T., Ikawa, S., Akiyama, T., Semba, K., Nomura, N., Miyajima, N., Saito, T., and Toyoshima, K. (1986) Nature 319, 230-234; Beerli, R. R., Segal, D. J., Dreier, B., and Barbas, C. F., III (1998) Proc. Natl. Acad. Sci. USA 95, 14628-14633]. The E2C-ER-VP64 fusion protein was constitutively active on a reporter carrying a single E2C binding site, almost as active as an E2C-VP64 fusion without an ER LBD, and did not respond well to hormone. Apparently, the use of a large DNA binding domain recognizing an extended stretch of DNA with high affinity renders the chimera hormone- and dimerization-independent.

To overcome these problems, we produced two types of ER-based chimeric regulators, designed to be capable of regulating gene expression through a single binding site in a hormone-dependent manner. In the first strategy, a heterodimeric regulator was generated consisting of the engineered zinc finger protein E2C fused to an ER LBD, as well as an ER LBD fused to a VP64 activation domain (Fig. 2A). When this heterodimeric regulator was expressed in HeLa cells, it had no significant activity on the E2C-TATA-lue reporter plasmid in the absence of 4-OHT. Addition of hormone led to a 3- to 5-fold stimulation of luciferase expression, indicating the formation of functional heterodimers. However, hormone-induced reporter gene activation was significantly lower than that induced by an E2C-VP64 fusion protein, presumably at least in part due to the formation of E2C-ER and ER-VP64 homodimers. Homodimers were inactive, since neither E2C-ER nor ER-VP64 alone induced luciferase expression. In the second strategy, fusion proteins were generated by combining the dimerization partners E2C-ER and ER-VP64 in one single polypeptide, through a flexible polypeptide linker. Two linkers were tested, 18 and 30 amino acids in length, creating the proteins E2C-scER/18-VP64 and E2C-scER/30-VP64 (Fig. 2A). These proteins were expected to be activated via intramolecular, rather than intermolecular, dimerization and therefore functional as monomers. Combination of two ER LBDs into one single-chain fusion construct should allow a more efficient hormone-induced dimerization and therefore yield more efficient activators. Indeed, when E2C-scER/18-VP64 and E2C-scER/30-VP64 were transiently expressed in HeLa cells, they efficiently activated the E2C-TATA-luc reporter in a largely hormone-dependent manner (Fig. 2B, 2C and 2D). Thus, dimeric regulators requiring response elements similar to those of natural steroid hormone receptors were successfully converted into monomeric, ligand-dependent transcription factors.

**Monomeric gene-switch based on EcR and RXR LBDs.** To show that the production of a ligand-dependent monomeric gene switch by fusion with two LBDs is a generally applicable strategy, the utility of other nuclear hormone receptors was tested. In particular, utility of the LBDs of the *Drosophila* ecdysone receptor (EcR) was investigated. In *Drosophila,* this receptor functions as a heterodimer between EcR and the product of the *ultraspiracle* (USP) gene [Yao, T.-P., Forman, B. M., Jiang, Z., Cherbas, L., Chen, J.-D., McKeown, M., Cherbas, P., and Evans, R. M. (1993) Nature366, 476-479]. However, it has been shown that EcR also efficiently heterodimerizes with USP's vertebrate homologue retinoid X receptor (RXR) in response to the ecdysone agonists Muristerone A or Ponasterone A (PonA) [Nakanishi, K. (1992) Steroids 57, 649-657; Yao, T.-P., Forman, B. M., Jiang, Z., Cherbas, L., Chen, J.-D., McKeown, M., Cherbas, P., and Evans, R. M. (1993) Nature 366, 476-479; No, D., Yao, T.-P., and Evans, R. M. (1996) Proc. Natl. Acad. Sci. USA 93, 3346-3351]. The EcR and RXR LBDs were therefore used to prepare a monomeric gene switch analogous to the scER chimeras described above (Fig. 3A). Thus, the human RXRα LBD (aa373-654) and the *Drosophila* EcR LBD (aa202-462) were inserted in between the E2C DBD and the VP64 activation domain, creating E2C-RE-VP64. In this fusion construct, the two LBDs are connected by an 18 amino acid flexible linker, the same that was used in E2C-scER/18-VP64. When this chimeric regulator was transiently expressed in HeLa cells along with the E2C-TATA-luc reporter plasmid, significant basal activity was observed. However, activity could be increased 3-fold by PonA, showing that this artificial construct was hormone-responsive. To improve the ligand dependence, the length of the linker connecting the RXR and EcR LBDs was increased, a measure that seemed beneficial in the case of the single-chain ER constructs. A longer linker should allow the LBDs to optimize their contact and add to the conformational disorder in the unliganded state. Indeed, when the linker was elongated to 30 aa (in E2C-RLE-VP64) or 36 aa (in E2C-RLLE-VP64), basal activity was significantly reduced and PonA led to a 9- to 10-fold activation, an extent of responsiveness comparable to the one of the single-chain ER fusion constructs (Fig. 3B). Thus, serial connection of pairs of nuclear hormone receptor LBDs appears to be a generally applicable strategy to render monomeric DNA binding proteins ligand-dependent.

The hPR and mER LBDs used for the fusion proteins did not encompass their natural SV40-like nuclear localization signals (NLS), located between amino acids 637 and 644 in hPR, and between amino acids 260 and 267 in mER [Carson-Jurica, M. A., Schrader, W. T., and O'Malley, W. (1990) Endocrine Reviews 11, 201-220]. While it has been shown that this NLS is not required for hormone-dependent nuclear localization of hPR, regulation of the subcellular localization of steroid receptors appears to be complex, and it was not *a priori* clear whether the presence of the SV40-like NLS was required for proper function of the chimeric proteins. Thus, additional constructs were prepared that incorporated an SV40 NLS (PKKKRKV) (SEQ ID NO: 30) in single letter amino acid code), either between VP16 and C7, or between C7 and LBD.

The chimeric transcriptional regulators were then tested for their ability to regulate the 10xC7-TATA-luc reporter plasmid in a hormone dependent manner. 10xC7-TATA-luc contains ten C7 binding sites [5'-GCG TGG GCG-3'] spaced by 5 nucleotides, and a TATA box, upstream of the firefly luciferase coding region. Each of the fusion proteins upregulated expression of luciferase in a largely hormone dependent manner. RU486 stimulated the activity of VP16-C7-PR 26-fold, while 4-OHT led to a 43-fold activation of VP16-C7-ER. There was no detectable crossreactivity between RU486 and ER, or between 4-OHT and PR. The presence of a NLS in either position was not only not required, but even undesirable, since it led to an increased basal (i.e. hormone-independent) activity of the fusion constructs, presumably through increased nuclear localization. Thus, the hPR (aa645-914) and mER (aa281-599, G525R) LBDs are able to confer hormone-dependence onto the zinc finger protein C7.

The ability to reversibly control the expression of multiple genes, or alleles of a gene, could prove very useful for many basic research applications. In particular, selective and independent expression of one gene, but not another (and *vice versa*), by small and nontoxic ligands would allow for a comparative analysis of gene function, both *in vitro* and *in vivo*. We have shown that our modular system for controlling target gene expression is indeed able to independently control the expression of two genes within the same transfected cell, as evidenced by RU486-dependent luciferase induction and 4-OHT-induced β-gal expression. The lack of β-gal induction by RU486, and luciferase induction by 4-GHT convincingly demonstrates the specificity of the chimeric regulators described here. Not only is the exquisite specificity of the utilized DNA binding domains retained, but also there is no detectable crossreaction between RU486 and the ER LBD, or between 4-OHT and the PR LBD.

## Claims

1. A non-naturally occurring polypeptide comprising two ligand binding domains derived from nuclear hormone receptors operatively linked to a first functional domain, wherein the first functional domain is a zinc finger nucleotide binding domain.

2. The polypeptide of claim 1 wherein the two ligand binding domains are covalently linked by means of a peptide linker.

3. The polypeptide of claim 2 wherein the linker contains from about 10 to about 40 amino acid residues.

4. The polypeptide of claim 2 wherein the linker contains from about 15 to about 35 amino acid residues.

5. The polypeptide of claim 2 wherein the linker contains from about 18 to about 30 amino acid residues.

6. The polypeptide of claim 1 wherein the first and second ligand binding domains are derived from different nuclear hormone receptors.

7. The polypeptide of claim 1 wherein the first and second ligand binding domains are derived from the same nuclear hormone receptor.

8. The polypeptide of claim 1 wherein the nuclear hormone receptor is an estrogen receptor, a progesterone receptor, an ecdysone receptor, a retinoic acid receptor, or a retinoid X receptor.

9. The polypeptide of claim 1 wherein at least one of the ligand binding domains is derived from a retinoid X receptor.

10. The polypeptide of claim 1 wherein the zinc finger nucleotide binding domain comprises at least one zinc finger DNA binding motif.

11. The polypeptide of claim 10 that comprises from two to twelve zinc finger DNA binding motifs.

12. The polypeptide of claim 10 that comprises from two to six zinc finger binding motifs.

13. The polypeptide of claim 10 wherein the zinc finger DNA binding motifs specifically bind to a nucleotide sequence of the formula (GNN)₁₋₆, where GNN specifies the base sequence of the nucleotide such that G is guanine and N is any possible base.

14. The polypeptide of claim 10 wherein the at least one zinc finger DNA binding motif is derived from a Cys₂-His₂ zinc finger.

15. A non-naturally occurring polypeptide comprising two ligand binding domains operatively linked to a first functional domain, wherein the first functional domain is selected from the group consisting of a zinc finger DNA binding domain and a nuclease domain.

16. The polypeptide of claim 15 wherein the two ligand binding domains are covalently linked by means of a peptide linker.

17. The polypeptide of claim 16 wherein the linker contains from about 10 to about 40 amino acid residues.

18. The polypeptide of claim 16 wherein the linker contains from about 15 to about 35 amino acid residues.

19. The polypeptide of claim 16 wherein the linker contains from about 18 to about 30 amino acid residues.

20. The polypeptide of claim 15 wherein the first functional domain is a zinc finger DNA binding domain.

21. The polypeptide of claim 20 wherein the zinc finger DNA binding domain comprises at least one zinc finger DNA binding motif.

22. The polypeptide of claim 21 that comprises from two to twelve DNA binding motifs.

23. The polypeptide of claim 21 that comprises from two to six DNA binding motifs.

24. The polypeptide of claim 21 wherein the zinc finger DNA binding motifs specifically bind to a nucleotide sequence of the formula (GNN)₁₋₆, where GNN specifies the base sequence of the nucleotide such that G is guanine and N is any possible base.

25. The polypeptide of claim 21 wherein the at least one zinc finger DNA binding motif is derived from a Cys₂-His₂ zinc finger.

26. The polypeptide of claim 15 further comprising a second functional domain operatively linked to either one of the ligand binding domains or the first functional domain.

27. The polypeptide of claim 26 wherein the first functional domain is a zinc finger DNA binding domain and the second functional domain is a transcriptional regulating domain.

28. The polypeptide of claim 27 wherein the DNA binding domain comprises at least one zinc finger DNA binding motif.

29. The polypeptide of claim 28 that comprises from two to twelve zinc finger DNA binding motifs.

30. The polypeptide of claim 28 that comprises from two to six zinc finger DNA binding motifs.

31. The polypeptide of claim 28 wherein the zinc finger DNA binding motifs specifically bind to a nucleotide sequence of the formula (GNN)₁₋₆, where GNN specifies the base sequence of the nucleotide such that G is guanine and N is any possible base.

32. The polypeptide of claim 28 wherein the at least one zinc finger DNA binding motif is derived from a Cys₂-His₂ zinc finger.

33. The polypeptide of claim 27 wherein the transcription regulating domain is an activation domain.

34. The polypeptide of claim 27 wherein the transcription regulating domain is a repression domain.

35. A non-naturally occurring polypeptide comprising two ligand binding domains operatively linked to a first functional domain, wherein the first functional domain is selected from the group consisting of a DNA binding domain and a nuclease domain, and wherein the first functional domain is separated from the ligand binding domains.

36. The polypeptide of claim 35 wherein the two ligand binding domains are covalently linked by means of a peptide linker.

37. The polypeptide of claim 36 wherein the linker contains from about 10 to about 40 amino acid residues.

38. The polypeptide of claim 36 wherein the linker contains from about 15 to about 35 amino acid residues.

39. The polypeptide of claim 36 wherein the linker contains from about 18 to about 30 amino acid residues.

40. The polypeptide of claim 35 further comprising a second functional domain operatively linked to either one of the ligand binding domains or the first functional domain.

41. The polypeptide of claim 1 or 15 wherein the zinc finger nucleotide binding domain is selected from the group consisting of:
(a) a zinc finger nucleotide binding domain selected from the group consisting of E2C, C7, B3B, 2C7, B3C2, and N1;
(b) a zinc finger nucleotide binding domain derived from a zinc finger nucleotide binding domain selected from the group consisting of E2C, C7, B3B, 2C7, B3C2, and N1 by truncation;
(c) a zinc finger nucleotide binding domain derived from a zinc finger nucleotide binding domain selected from the group consisting of E2C, C7, B3B, 2C7, B3C2, and N 1 by expansion; and
(d) a zinc finger nucleotide binding domain derived from a zinc finger nucleotide binding domain selected from the group consisting of E2C, C7, B3B, 2C7, B3C2, and N1 by mutagenesis.

42. The polypeptide of claim 33 wherein the activation domain is selected from the group consisting of VP16, TA2, VP64, STAT6, relA, TAF-1, TAF-2, TAU-1, and TAU-2.

43. The polypeptide of claim 34 wherein the repression domain is selected from the group consisting of ERD, KRAB, SID, histone deacetylase, DNA methylase, and a derivative or multimer of KRAB, SID, or ERD selected from the group consisting of KRAB-ERD, SID-ERD, (KRAB)₂, (KRAB)₃, KRAB-A, (KRAB-A)₂, (SID)₂, (KRAB-A)-SID, and SID-KRAB-A.

44. The polypeptide of claim 1 or 15 wherein at least one of the ligand binding domains binds a ligand selected from the group consisting of: 11β-(4-dimethylaminophenyl)-17β-hydroxy-17α-propinyl-4,9-estradiene-3-one; 11β-(4-dimethylaminophenyl)-17α-hydroxy-17β-(3-hydroxypropyl)-13α-methyl-4,9-gonadiene-3-one; 11β-(4-acetylphenyl)-17β-hydroxy-17α-(1-propinyl)-4,9-estradiene-3-one; 11β-(4-dimethylaminophenyl)-17β-hydroxy-17α-(3-hydroxy-1(Z)-propenyl-estra-4,9-diene-3-one ;(7β, 11β, 17β)11-(4-dimethylaminophenyl)-7-methyl-4',5'-dihydrospiro[ester-4,9-diene-17,2' (3'H)-furan]-3-one; (11β,14β,17α)-4',5'-dihydro-11-(4-dimethylaminophenyl)[spiroestra-4,9-diene-17,2' (3'H)-furan]-3-one; and 5-alpha-pregnane-3,2-dione.

45. The polypeptide of claim 1 wherein at least one of the nuclear receptors is selected from the group consisting of estrogen receptor (ER), progesterone receptor (PR), glucocorticoid-α receptor, glucocorticoid-β receptor, mineralocorticoid receptor, androgen receptor, thyroid hormone receptor, retinoic acid receptor (RAR), retinoid X receptor (RXR), Vitamin D receptor, COUP-TF receptor, ecdysone receptor (EcR), Nurr-1 receptor, and orphan receptors.

46. The polypeptide of claim 1 wherein the polypeptide is a gene switch using RXR, E2C, and activation domains wherein the gene switch is selected from the group consisting of E2C-RXR-L-DE-VP64, E2C-RXR-LL-DE-VP64, E2C-RXR-LLL-DE-VP64, E2C-RXR-L-BE-VP64, EZC-RXR-LL-BE-VP64, E2C-RXR-LLL-BE-VP64, E2C-RXR-L-DE-VP 16, E2C-RXR-LL-DE-VP 16, E2C-RXR-LLL-DE-VP 16, E2C-RXR-L-BE-VP 16, E2C-RXR-LL-BE-VP 16, and E2C-RXR-LLL-BE-VP16.

47. The polypeptide of claim 1 wherein the polypeptide is a gene switch using RXR, 2C7, and activation domains wherein the gene switch is selected from the group consisting of2C7-RXR-L-DE-VP64, 2C7-RXR-LL-DE-VP64, 2C7-RXR-LLL-DE-VP64, 2C7-RXR-L-BE-VP64, 2C7-RXR-LL-BE-VP64, 2C7-RXR-LLL-BE-VP64, 2C7-RXR-L-DE-VP16, 2C7-RXR-LL-DE-VP16, 2C7-RXR-LLL-DE-VP16, 2C7-RXR-L-BE-VP16, 2C7-RXR-LL-BE-VP16, and E2C-RXR-LLL-BE-VP16.

48. The polypeptide of claim 1 wherein the polypeptide is a gene switch using RXR, B3B, and activation domains wherein the gene switch is selected from the group consisting of B3B-RXR-L-DE-VP64, B3B-RXR-LL-DE-VP64, B3B-RXR-LLL-DE-VP64, B3B 7-RXR-L-BE-VP64, B3B 7-RXR-LL-BE-VP64, B3B-RXR-LLL-BE-VP64, B3B-RXR-L-DE-VP16, B3B-RXR-LL-DE-VP16, B3B-RXR-LLL-DE-VP16, B3B-RXR-L-BE-VP16, B3B-RXR-LL-BE-VP16, and B3B-RXR-LLL-BE-VP16.

49. The polypeptide of claim 1 wherein the polypeptide is a gene switch using RXR, B3C2, and activation domains wherein the gene switch is selected from the group consisting of B3C2-RXR-L-DE-VP64, B3C2-RXR-LL-DE-VP64, B3C2-RXR-LLL-DE-VP64, B3C2-RXR-L-BE-VP64, B3C2-RXR-LL-BE-VP64, B3C2-RXR-LLL-BE-VP64, B3C2-RXR-L-DE-VP16, B3C2-RXR-LL-DE-VP16, B3C2-RXR-LLL-DE-VP16, B3C2-RXR-L-BE-VP16, B3C2 B-RXR-LL-BE-VP16, and B3C2-RXR-LLL-BE-VP 16.

50. The polypeptide of claim 1 wherein the polypeptide is a gene switch using RXR, E2C, and repression domains wherein the gene switch is selected from the group consisting of E2C-RXR-L-DE-(KRAB-A)₂, E2C-RXR-LL-DE-(KRAB-A)₂, E2C-RXR-LLL-DE-(KRAB-A)₂, E2C-RXR-L-BE-(KRAB-A)₂, E2C-RXR-LL-BE-(KRAB-A)₂, E2C-RXR-LLL-BE-(KRAB-A)₂, E2C-RXR-L-DE-(KRAB-A)₂, E2C-RXR-LL-DE-(KRAB-A)₂, E2C-RXR-LLL-DE -(KRAB-A)₂, E2C-RXR-L-BE-(KRAB-A)₂, E2C-RXR-LL-BE-(KRAB-A)₂, E2C-RXR-LLL-BE-(KRAB-A)₂, E2C-RXR-L-DE-(SID)₂, E2C-RXR-LL-DE-(SID)₂, E2C-RXR-LLL-DE-(SID)₂, E2C-RXR-L-BE-(SID)₂, E2C-RXR-LL-BE-(SID)₂, E2C-RXR-LLL-BE-(SID)₂, E2C-RXR-L-DE-(SID)₂, E2C-RXR-LL-DE-(SID)₂, E2C-RXR-LLL-DE-(SID)₂, E2C-RXR-L-BE-(SID)₂, E2C-RXR-LL-BE-(SID)₂, and E2C-RXR-LLL-BE-(SID)₂.

51. The polypeptide of claim 1 wherein the polypeptide is a gene switch using RXR, 2C7, and repression domains wherein the gene switch is selected from the group consisting of 2C7-RXR-L-DE-(KRAB-A)₂, 2C7-RXR-LL-DE-(KRAB-A)₂, 2C7-RXR-LLL-DE-(KRAB-A)₂, 2C7-RXR-L-BE-(KRAB-A)₂, 2C7-RXR-LL-BE-(KRAB-A)₂, 2C7-RXR-LLL-BE-(KRAB-A)₂, 2C7-RXR-L-DE-(KRAB-A)₂, 2C7-RxR-LL-DE-(KRAB-A)₂, 2C7-RXR-LLL-DE-(KRAB-A)₂, 2C7-RXR-L-BE-(KRAB-A)₂, 2C7-RXR-LL-BE-(KRAB-A)₂, E2C-RXR-LLL-BE-(KRAB-A)₂, 2C7-RXR-L-DE-(SID)₂, 2C7-RXR-LL-DE-(SID)₂, 2C7-RXR-LLL-DE-(SID)₂, 2C7-RXR-L-BE-(SID)₂, 2C7-RXR-LL-BE-(SID)₂, 2C7-RXR-LLL-BE-(SID)₂, 2C7-RXR-L-DE-(SID)₂, 2C7-RXR-LL-DE-(SID)₂, 2C7-RXR-LLL-DE-(SID)₂, 2C7-RXR-L-BE-(SID)₂, 2C7-RXR-LL-BE-(SID)₂, and E2C-RXR-LLL-BE-(SID)₂.

52. The polypeptide of claim 1 wherein the polypeptide is a gene switch using RXR, B3B, and repression domains wherein the gene switch is selected from the group consisting of B3B-RXR-L-DE-(KRAB-A)₂, B3B-RXR-LL-DE-(KRAB-A)₂, B3B-RXR-LLL-DE-(KRAB-A)₂, B3B 7-RXR-L-BE-(KRAB-A)₂, B3B 7-RXR-LL-BE-(KRAB-A)₂, B3B-RXR-LLL-BE-(KRAB-A)₂, B3B-RXR-L-DE-(KRAB-A)₂, B3B-RXR-LL-DE-(KRAB-A)₂, B3B-RXR-LLL-DE-(KRAB-A)₂, B3B-RXR-L-BE-(KRAB-A)₂, B3B-RXR-LL-BE-(KRAB-A)₂, B3B-RXR-LLL-BE-(KRAB-A)₂, B3B-RXR-L-DE-(SID)₂, B3B-RXR-LL-DE-(SID)₂, B3B-RXR-LLL-DE-(SID)₂, B3B 7-RXR-L-BE-(SID)₂, B3B 7-RXR-LL-BE-(SID)₂, B3B-RXR-LLL-BE-(SID)₂, B3B-RXR-L-DE-(SID)₂, B3B-RXR-LL-DE-(SID)₂, B3B-RXR-LLL-DE-(SID)₂, B3B-RXR-L-BE-(SID)₂, B3B-RXR-LL-BE-(SID)₂, B3B-RXR-LLL-BE-(SID)₂.

53. The polypeptide of claim 1 wherein the polypeptide is a gene switch using RXR, B3C2, and repression domains wherein the gene switch is selected from the group consisting of B3C2RXR-L-DE-(KRAB-A)₂, B3 C2-RXR-LL-D-(KRAB-A₎₂, B3C2-RXR-LLL-DE-(KRAB-A)₂, B3C2-RXR-L-BE-(KRAB-A)₂, B3C2-RXR-LL-BE-(KRAB-A)₂, B3C2-RXR-LLL-BE-(KRAB-A)₂, B3C2-RXR-L-DE-(KRAB-A)₂, B3C2-RXR-LL-DE-(KRAB-A)₂, B3C2-RXR-LLL-DE-(KRAB-A)₂, B3C2-RXR-L-BE-(KRAB-A)₂, B3C2 B-RXR-LL-BE-(KRAB-A)₂, B3C2-RXR-LLL-BE-(KRAB-A)₂, B3C2-RXR-L-DE-(SID)₂, B3C2-RXR-LL-DE-(SID)₂, B3C2-RXR-LLL-DE-(SID)₂, B3 C2-RXR-L-BE-(SID)₂ , B3C2-RXR-LL-BE-(SID)₂, B3C2-RXR-LLL-BE -(SID)₂, B3C2-RXR-L-DE-(SID)₂, B3C2-RXR-LL-DE-(SID)₂, B3C2-RXR-LLL-DE-(SID)₂, B3C2-RXR-L-BE-(SID)₂, B3C2 B-RXR-LL-BE-(SID)₂, and B3C2-RXR-LLL-BE-(SID)₂.

54. The polypeptide of claim 1 wherein the polypeptide is a gene switch using PR, E2C, and activation domains wherein the gene switch is selected from the group consisting of E2C-PR-L-PR-VP64, E2C-PR-LL-PR-VP64, E2C-PR-LLL-PR-VP64, E2C-PR-L-PR-VP64, E2C-PR-LL-PR-VP64, E2C-PR-LLL-PR-VP64, E2C-PR-L-PR-VP 16, E2C-PR-LL-PR-VP 16, E2C-PR-LLL-PR-VP 16, E2C-PR-L-PR-VP16, E2C-PR-LL-PR-VP16, and E2C-PR-LLL-PR-VP16.

55. The polypeptide of claim 1 wherein the polypeptide is a gene switch using PR, 2C7, and activation domains wherein the gene switch is selected from the group consisting of 2C7-PR-L-PR-VP64, 2C7-PR-LL-PR-VP64, 2C7-PR-LLL-PR-VP64, 2C7-PR-L-PR-VP64, 2C7-PR-LL-PR-VP64, 2C7-PR-LLL-PR-VP64, 2C7-PR-L-PR-VP16, 2C7-PR-LL-PR-VP16, 2C7-PR-LLL-PR-VP16, 2C7-PR-L-PR-VP16,2C7-PR-LL-PR-VP16, and E2C-PR-LLL-PR-VP16.

56. The polypeptide of claim 1 wherein the polypeptide is a gene switch using PR, B3B, and activation domains wherein the gene switch is selected from the group consisting of B3B-PR-L-PR-VP64, B3B-PR-LL-PR-VP64, B3B-PR-LLL-PR-VP64, B3B 7-PR-L-PR-VP64, B3B 7-PR-LL-PR-VP64, B3B-PR-LLL-PR-VP64, B3B-PR-L-PR-VP16, B3B-PR-LL-PR-VP16, B3B-PR-LLL-PR-VP16, B3B-PR-L-PR-VP16, B3B-PR-LL-PR-VP16, and B3B-PR-LLL-PR-VP16.

57. The polypeptide of claim 1 wherein the polypeptide is a gene switch using PR, B3C2, and activation domains wherein the gene switch is selected from the group consisting of B3C2-PR-L-PR-VP64, B3C2-PR-LL-PR-VP64, B3C2-PR-LLL-PR-VP64, B3C2-PR-L-PR-VP64, B3C2-PR-LL-PR-VP64, B3C2-PR-LLL-PR-VP64, B3C2-PR-L-PR-VP16, B3C2-PR-LL-PR-VP16, B3C2-PR-LLL-PR-VP16, B3C2-PR-L-PR-VP16, B3C2 B-PR-LL-PR-VP16, and B3C2-PR-LLL-PR-VP16.

58. The polypeptide of claim 1 wherein the polypeptide is a gene switch using PR, E2C, and repression domains wherein the gene switch is selected from the group consisting of E2C-PR-L-PR-(KRAB-A)₂, E2C-PR-LL-PR-(KRAB-A)₂, E2C-PR-LLL-PR-(KRAB-A)₂, E2C-PR-L-PR-(KRAB-A)₂, E2C-PR-LL-PR-(KRAB-A)₂, E2C-PR-LLL-PR-(KRAB-A)₂, E2C-PR-L-PR-(KRAB-A)₂, E2C-PR-LL-PR-(KRAB-A)₂, E2C-PR-LLL-PR-(KRAB-A)₂, E2C-PR-L-PR-(KRAB-A)₂, E2C-PR-LL-PR-(KRAB-A)₂, E2C-PR-LLL-PR-(KRAB-A)₂, E2C-PR-L-PR-(SID)₂, E2C-PR-LL-PR-(SID)₂, E2C-PR-LLL-PR-(SID)₂, E2C-PR-L-PR-(SID)₂, E2C-PR-LL-PR-(SID)₂, E2C-PR-LLL-PR-(SID)₂, E2C-PR-L-PR-(SID)₂, E2C-PR-LL-PR-(SID)₂, E2C- PR-LLL-PR-(SID)₂, E2C- PR-L-PR-(SID)₂, E2C-PR-LL-PR-(SID)₂, and E2C-PR-LLL-PR-(SID)₂.

59. The polypeptide of claim 1 wherein the polypeptide is a gene switch using PR, 2C7, and repression domains wherein the gene switch is selected from the group consisting of 2C7-PR-L-PR-(KRAB-A)₂, 2C7-PR-LL-PR-(KRAB-A)₂, 2C7-PR-LLL-PR-(KRAB-A)₂, 2C7-PR-L-PR-(KRAB-A)₂, 2C7-PR-LL-PR-(KRAB-A)₂, 2C7-PR-LLL-PR-(KRAB-A)₂, 2C7-PR-L-PR-(KRAB-A)₂, 2C7-PR-LL-PR-(KRAB-A)₂, 2C7-PR-LLL-PR-(KRAB-A)₂, 2C7-PR-L-PR-(KRAB-A)₂, 2C7-PR-LL-PR-(KRAB-A)₂, E2C-PR-LLL-PR-(KRAB-A)₂, 2C7-PR-L-PR-(SID)₂, 2C7-PR-LL-PR-(SID)₂, 2C7-PR-LLL-PR-(SID)₂, 2C7-PR-L-PR-(SID)₂, 2C7-PR-LL-PR-(SID)₂, 2C7-PR-LLL-PR-(SID)₂, 2C7-PR-L-PR-(SID)₂, 2C7-PR-LL-PR-(SID)₂, 2C7-PR-LLL-PR-(SID)₂, 2C7-PR-L-PR-(SID)₂, 2C7-PR-LL-PR-(SID)₂, and E2C-PR-LLL-PR-(SID)₂.

60. The polypeptide of claim 1 wherein the polypeptide is a gene switch using PR, B3B, and repression domains wherein the gene switch is selected from the group consisting of B3B-PR-L-PR-(KRAB-A)₂, B3B-PR-LL-PR-(KRAB-A)₂, B3B-PR-LLL-PR-(KRAB-A)₂, B3B 7-PR-L-PR-(KRAB-A)₂, B3B 7-PR-LL-PR-(KRAB-A)₂, B3B-PR-LLL-PR-(KRAB-A)₂, B3B-PR-L-PR-(KRAB-A)₂, B3B-PR-LL-PR-(KRAB-A)₂, B3B-PR-LLL-PR-(KRAB-A)₂, B3B-PR-L-PR-(KRAB-A)₂, B3B-PR-LL-PR-(KRAB-A)₂, B3B-PR-LLL-PR-(KRAB-A)₂, B3B-PR-L-PR-(SID)₂, B3B-PR-LL-PR-(SID)₂, B3B-PR-LLL-PR-(SID)₂, B3B 7-PR-L-PR-(SID)₂, B3B7-PR-LL-PR-(SID)₂, B3B-PR-LLL-PR-(SID)₂, B3B-PR-L-PR-(SID)₂, B3B-PR-LL-PR-(SID)₂, B3B-PR-LLL-PR-(SID)₂, B3B-PR-L-PR-(SID)₂, B3B-PR-LL-PR-(SID)₂, and B3B-PR-LLL-PR-(SID)₂.

61. The polypeptide of claim 1 wherein the polypeptide is a gene switch using PR, B3C2, and repression domains wherein the gene switch is selected from the group consisting of B3C2-PR-L-PR-(KRAB-A)₂, B3C2-PR-LL-PR-(KRAB-A)₂, B3C2-PR-LLL-PR-(KRAB-A)₂, B3C2-PR-L-PR-(KRAB-A)₂, B3C2-PR-LL-PR-(KRAB-A)₂, B3C2-PR-LLL-PR-(KRAB-A)₂, B3C2-PR-L-PR-(KRAB-A)₂, B3C2-PR-LL-PR-(KRAB-A)₂, B3C2-PR-LLL-PR-(KRAB-A)₂, B3C2-PR-L-PR-(KRAB-A)₂, B3C2 B-PR-LL-PR-(KRAB-A)₂, B3C2-PR-LLL-PR-(KRAB-A)₂, B3C2-PR-L-PR-(SID)₂, B3C2-PR-LL-PR-(SID)₂, B3C2-PR-LLL-PR-(SID)₂, B3C2-PR-L-PR-(SID)₂, B3C2-PR-LL-PR-(SID)₂, B3C2-PR-LLL-PR-(SID)₂, B3C2-PR-L-PR-(SID)₂, B3C2-PR-LL-PR-(SID)₂, B3C2-PR-LLL-PR-(SID)₂, B3C2-PR-L-PR-(SID)₂, B3C2 B-PR-LL-PR-(SID)₂, and B3C2-PR-LLL-PR-(SID)₂.

62. The polypeptide of claim 1 wherein the polypeptide is a gene switch using ER, E2C, and activation domains wherein the gene switch is selected from the group consisting of E2C-ER-L-ER-VP64, E2C-ER-LL-ER-VP64, E2C-ER-LLL-ER-VP64, E2C-ER-L-ER-VP64, E2C-ER-LL-ER-VP64, E2C-ER-LLL-ER-VP64, E2C-ER-L-ER-VP16, E2C-ER-LL-ER-VP16, E2C-ER-LLL-ER-VP16, E2C-ER-L-ER-VP16, E2C-ER-LL-ER-VP16, and E2C-ER-LLL-ER-VP16.

63. The polypeptide of claim 1 wherein the polypeptide is a gene switch using ER, 2C7, and activation domains wherein the gene switch is selected from the group consisting of 2C7-ER-L-ER-VP64, 2C7-ER-LL-ER-VP64, 2C7-ER-LLL-ER-VP64, 2C7-ER-L-ER-VP64, 2C7-ER-LL-ER-VP64, 2C7-ER-LLL-ER-VP64, 2C7-ER-L-ER-VP16, 2C7-ER-LL-ER-VP16, 2C7-ER-LLL-ER-VP16, 2C7-ER-L-ER-VP16, 2C7-ER-LL-ER-VP16, and E2C-ER-LLL-ER-VP16.

64. The polypeptide of claim 1 wherein the polypeptide is a gene switch using ER, B3B, and activation domains wherein the gene switch is selected from the group consisting of B3B-ER-L-ER-VP64, B3B-ER-LL-ER-VP64, B3B-ER-LLL-ER-VP64, B3B 7-ER-L-ER-VP64, B3B 7-ER-LL-ER-VP64, B3B-ER-LLL-ER-VP64, B3B-ER-L-ER-VP16, B3B-ER-LL-ER-VP16, B3B-ER-LLL-ER-VP16, B3B-ER-L-ER-VP16, B3B-ER-LL-ER-VP16, and B3B-ER-LLL-ER-VP16.

65. The polypeptide of claim 1 wherein the polypeptide is a gene switch using ER, B3C2, and activation domains wherein the gene switch is selected from the group consisting of B3C2-ER-L-ER-VP64, B3C2-ER-LL-ER-VP64, B3C2-ER-LLL-ER-VP64, B3C2-ER-L-ER-VP64, B3C2-ER-LL-ER-VP64, B3C2-ER-LLL-ER-VP64, B3C2-ER-L-ER-VP16, B3C2-ER-LL-ER-VP16, B3C2-ER-LLL-ER-VP16, B3C2-ER-L-ER-VP16, B3C2 B-ER-LL-ER-VP16, and B3C2-ER-LLL-ER-VP16.

66. The polypeptide of claim 1 wherein the polypeptide is a gene switch using ER, E2C, and repression domains wherein the gene switch is selected from the group consisting of E2C-ER-L-ER-(KRAB-A)₂, E2C-ER-LL-ER-(KRAB-A)₂, E2C-ER-LLL-ER-(KRAB-A)₂, E2C-ER-L-ER-(KRAB-A)₂, E2C-ER-LL-ER-(KRAB-A)₂, E2C-ER-LLL-ER-(KRAB-A)₂, E2C-ER-L-ER-(KRAB-A)₂, E2C-ER-LL-ER-(KRAB-A)₂, E2C-ER-LLL-ER-(KRAB-A)₂, E2C-ER-L-ER-(KRAB-A)₂, E2C-ER-LL-ER-(KRAB-A)₂, E2C-ER-LLL-ER-(KRAB-A)₂, E2C-ER-L-ER-(SID)₂, E2C-ER-LL-ER-(SID)₂, E2C-ER-LLL-ER-(SID)₂, E2C-ER-L-ER-(SID)₂, E2C-ER-LL-ER-(SID)₂, E2C-ER-LLL-ER-(SID)₂, E2C-ER-L-ER-(SID)₂, E2C-ER-LL-ER-(SID)₂, E2C-ER-LLL-ER-(SID)₂, E2C-ER-L-ER-(SID)₂, E2C-ER-LL-ER-(SID)₂, and E2C-ER-LLL-ER-(SID)₂.

67. The polypeptide of claim 1 wherein the polypeptide is a gene switch using ER, 2C7, and repression domains wherein the gene switch is selected from the group consisting of 2C7-ER-L-ER-(KRAB-A)₂, 2C7-ER-LL-ER-(KRAB-A)₂, 2C7-ER-LLL-ER-(KRAB-A)₂, 2C7-ER-L-ER-(KRAB-A)₂, 2C7-ER-LL-ER-(KRAB-A)₂, 2C7-ER-LLL-ER-(KRAB-A)₂, 2C7-ER-L-ER-(KRAB-A)₂, 2C7-ER-LL-ER-(KRAB-A)₂, 2C7-ER-LLL-ER-(KRAB-A)₂, 2C7-ER-L-ER-(KRAB-A)₂, 2C7-ER-LL-ER-(KRAB-A)₂, E2C-ER-LLL-ER-(KRAB-A)₂, 2C7-ER-L-ER-(SID)₂, 2C7-ER-LL-ER-(SID)₂, 2C7-ER-LLL-ER-(SID)₂, 2C7-ER-L-ER-(SID)₂,2C7-ER-LL-ER-(SID)₂, 2C7-ER-LLL-ER-(SID)₂, 2C7-ER-L-ER-(SID)₂, 2C7-ER-LL-ER-(SID)₂, 2C7-ER-LLL-ER-(SID)₂, 2C7-ER-L-ER-(SID)₂, 2C7-ER-LL-ER-(SID)₂, and E2C-ER-LLL-ER-(SID)₂.

68. The polypeptide of claim 1 wherein the polypeptide is a gene switch using ER, B3B, and repression domains wherein the gene switch is selected from the group consisting of B3B-ER-L-ER-(KRAB-A)₂, B3B-ER-LL-ER-(KRAB-A)₂, B3B-ER-LLL-ER-(KRAB-A)₂, B3B 7-ER-L-ER-(KRAB-A)₂, B3B 7-ER-LL-ER-(KRAB-A)₂, B3B-ER-LLL-ER-(KRAB-A)₂, B3B-ER-L-ER-(KRAB-A)₂, B3B-ER-LL-ER-(KRAB-A)₂, B3B-ER-LLL-ER-(KRAB-A)₂, B3B-ER-L-ER-(KRAB-A)₂, B3B-ER-LL-ER-(KRAB-A)₂, B3B-ER-LLL-ER-(KRAB-A)₂, B3B-ER-L-ER-(SID)₂, B3B-ER-LL-ER-(SID)₂, B3B-ER-LLL-ER-(SID)₂, B3B 7-ER-L-ER-(SID)₂, B3B 7-ER-LL-ER-(SID)₂, B3B-ER-LLL-ER-(SID)₂, B3B-ER-L-ER-(SID)₂, B3B-ER-LL-ER-(SID)₂, B3B-ER-LLL-ER-(SID)₂, B3B-ER-L-ER-(SID)₂, B3B-ER-LL-ER-(SID)₂, and B3B-ER-LLL-ER-(SID)₂.

69. The polypeptide of claim 1 wherein the polypeptide is a gene switch using ER, B3C2, and repression domains wherein the gene switch is selected from the group consisting of B3C2-ER-L-ER-(KRAB-A)₂, B3C2-ER-LL-ER-(KRAB-A)₂, B3C2-ER-LLL-ER-(KRAB-A)₂, B3C2-ER-L-ER-(KRAB-A)₂, B3C2-ER-LL-ER-(KRAB-A)₂, B3C2-ER-LLL-ER-(KRAB-A)₂, B3C2-ER-L-ER-(KRAB-A)₂, B3C2-ER-LL-ER-(KRAB-A)₂, B3C2-ER-LLL-ER-(KRAB-A)₂, B3C2-ER-L-ER-(KRAB-A)₂, B3C2 B-ER-LL-ER-(KRAB-A)₂, B3C2-ER-LLL-ER-(KRAB-A)₂, B3C2-ER-L-ER-(SID)₂, B3C2-ER-LL-ER-(SID)₂, B3C2-ER-LLL-ER-(SID)₂, B3C2-ER-L-ER-(SID)₂, B3C2-ER-LL-ER-(SID)₂, B3C2-ER-LLL-ER-(SID)₂, B3C2-ER-L-ER-(SID)₂, B3C2-ER-LL-ER-(SID)₂, B3C2-ER-LLL-ER-(SID)₂, B3C2-ER-L-ER-(SID)₂, B3C2 B-ER-LL-ER-(SID)₂, and B3C2-ER-LLL-ER-(SID)₂.

70. The polypeptide of claim 1 or 15 wherein the polypeptide has activation or repression activity at multiple promoters.

71. A polynucleotide that encodes the polypeptide of claim 1.

72. A polynucleotide that encodes the polypeptide of claim 15.

73. An expression vector comprising the polynucleotide of claim 71.

74. An expression vector comprising the polynucleotide of claim 72.

75. A cell containing the polynucleotide of claim 71.

76. A cell containing the polynucleotide of claim 72.

77. A host cell transformed with the expression vector of claim 73.

78. A host cell transformed with the expression vector of claim 74.

79. A process of regulating the function of a target nucleotide that contains a defined sequence, the process comprising exposing the target nucleotide to the polypeptide of claim 1 in the presence of a ligand that binds one of the ligand binding domains of the polypeptide, wherein the DNA binding domain of the polypeptide binds the defined sequence.

80. A process of regulating the function of a target nucleotide that contains a defined sequence, the process comprising exposing the target nucleotide to the polypeptide of claim 15 in the presence of a ligand that binds one of the ligand binding domain of the polypeptide, wherein the functional domain of the polypeptide alters the function of the target nucleotide.
